# EUROPEAN PATENT SPECIFICATION

(11) **EP 4 598 459 B1**
(45) Date of publication and mention of the grant of the patent: **25.11.2026**
(21) Application number: 23801612.5
(22) Date of filing: 03.10.2023
(51) Int. Cl.: A61B 17/3203

(54) **THROMBECTOMY SYSTEM INCLUDING A ROLLER PUMP ACTUATED VALVE**
THROMBEKTOMIESYSTEM MIT EINEM ROLLENPUMPENBETÄTIGTEN VENTIL
SYSTÈME DE THROMBECTOMIE COMPRENANT UNE SOUPAPE ACTIONNÉE PAR POMPE À ROULEAUX

(30) Priority: 04.10.2022 US 202263413054 P; 28.03.2023 US 202363455182 P
(43) Date of publication of application: 13.08.2025
(73) Proprietor: Boston Scientific Scimed, Inc., Maple Grove, MN 55311 (US)
(72) Inventor: FARAGO, Laszlo Trent, Hudson, Wisconsin 54016 (US); SCHROM, Michael P., Forest Lake, Minnesota 55025 (US); KUMAR, Ajay, Hisar, Haryana 125 005 (IN); CHANDRA, Sanjay Kumar, Pune 411 061 (IN); PAHWA, Rupesh Kumar, Gurugram 122 018 (IN)
(74) Representative: Vossius & Partner Patentanwälte Rechtsanwälte mbB
(86) International application number: PCT/US2023/075806
(87) International publication number: WO 2024/076970

(56) References cited:
- WO-A1-2015/196156
- US-A1- 2004 049 149
- US-A1- 2008 243 153
- US-A1- 2019 381 223
- US-B1- 10 531 883
- US-B2- 7 935 077

## Description

### CROSS-REFERENCE TO RELATED APPLICATIONS

This application claims the benefit of priority to U.S. Provisional Application Serial No. 63/413,054, filed October 4, 2022 and to U.S. Provisional Application Serial No. 63/455,182, filed March 28, 2023.

### TECHNICAL FIELD

The disclosure is directed to thrombectomy systems. More particularly, the disclosure is directed to a valve, such as a spool valve, for controlling a flow of effluent through a thrombectomy system from a patient.

### BACKGROUND

Thrombectomy is a procedure for removing thrombus from the vasculature of a patient. Mechanical and fluid based systems can be used to remove thrombus. With fluid based systems, an infusion fluid may be infused to a treatment area of a vessel with a catheter to dislodge the thrombus. In some instances, an effluent (e.g., the infusion fluid and/or blood) including the dislodged thrombus may be extracted from the vessel through the catheter. Of the known thrombectomy systems and methods, there is an ongoing need to provide alternative configurations of thrombectomy catheters and systems, as well as methods of operating such thrombectomy systems.

US 7 935 077 B2 relates to a thrombectomy catheter deployment system which includes a stand alone drive unit and a disposable pump/catheter assembly which is manually placed into a carriage assembly in the drive unit. The pump/catheter assembly has a plurality of preconnected components including tubular structure and a thrombectomy catheter connected thereto and is transported into or out of the interior of the drive unit for automatic high pressure pump piston head engagement or disengagement with a reciprocating linear actuator and for automatic engagement or disengagement of an effluent waste tube with a roller pump. A barcode reader senses specific operational data pertaining to an individual pump and provides an interface for operation of the reciprocating linear actuator. A bubble trap incorporated for effective debubbling of saline solution is closely coupled to an insert molded pump having an improved valve arrangement. A spiked bag with large tubing is incorporated for bubble-free transfer of saline.

US 2004/049149 A1 relates to a device for removing a thrombus or other tissue deposit from the cardiovascular system, or from a natural or synthetic tubule or other cavity found in the human body of a patient without the need to surgically access the location of the thrombus or other tissue deposit via a cut-down or other surgical procedure. A flexible metal or high pressure plastic tube conveys an extremely high pressure stream of sterile saline or other physiologic solution to at least one orifice at the distal end of the catheter. At least one jet is directed at the opening of a large exhaust lumen or other target. The jet is responsible for providing a localized negative pressure which entrains tissue into the jet from break-up of the debris. This jet can also provide stagnation pressure in the exhaust lumen which drives the tissue or thrombotic debris out of the exhaust lumen. The rate of exhaust of tissue debris is metered to ensure minimal local impact on the vasculature at the site of the thrombus deposit. A fluid metering means, such as a roller pump, controls the rate of exhaust such that it is in balance with the saline input or can be adjusted to be greater or less than the input.

US 10 531 883 B1 relates to a thrombectomy removal system which includes a catheter having a distal end and defining a lumen filled with a liquid column having a proximal portion and a distal portion, a vacuum source, a vent liquid source, and a vacuum and vent control system configured to cyclically connect or disconnect the vacuum source and the vent liquid source to change a level of vacuum at the distal end and substantially prevent forward flow. The vacuum source and the vent source are respectively fluidically connected to the vacuum and vent valves. A manifold connects the proximal portion to the vacuum and vent sources through the valves. Control of the valves prevents forward flow of the distal portion out from the distal end during each cycle. Each cycle can have states including vacuum-only, first double-closed, vent-only, and second double-closed. A time of the double-closed states can be no greater than 30 ms.

### SUMMARY

The invention is defined in the appended claims. Aspects, embodiments and examples disclosed herein which do not fall within the scope of the appended claims do not form part of the invention and are merely provided for illustrative purposes.

This disclosure provides design, material, manufacturing method, and use alternatives for medical devices.

In a first example, a thrombectomy catheter and pump assembly may comprise a thrombectomy catheter, a pump, a connection manifold assembly positioned between the thrombectomy catheter and the pump, an effluent return tube fluidly coupled to the thrombectomy catheter and the connection manifold assembly, an effluent collection bag, an effluent waste tube fluidly coupled to the connection manifold assembly and the effluent collection bag, and a valve positioned in line with the effluent waste tube.

Alternatively or additionally to any of the examples above, in another example, the valve may be configured to selectively allow a flow of effluent from the thrombectomy catheter to the effluent collection bag during use of the thrombectomy catheter.

Alternatively or additionally to any of the examples above, in another example, the valve may be a spool valve comprising a valve body defining an interior lumen and a spool movably disposed within the interior lumen.

Alternatively or additionally to any of the examples above, in another example, the spool may further comprise an annular groove extending about a circumference of the spool, the annular groove positioned between a first end of the spool and a second end of the spool

Alternatively or additionally to any of the examples above, in another example, the spool valve may include an effluent inlet port fluidly coupled to the effluent waste tube downstream of the connection manifold assembly and an effluent outlet port fluidly coupled to the effluent waste tube upstream of the effluent collection bag.

Alternatively or additionally to any of the examples above, in another example, the effluent inlet port and the effluent outlet port may be positioned on opposite sides of the valve body.

Alternatively or additionally to any of the example above, in another example, the valve further comprises first and second O-rings positioned between an outer surface of the spool and an inner surface of the valve body, wherein when the valve is in a closed configuration the effluent inlet port is positioned between the first and second O-rings.

Alternatively or additionally to any of the examples above, in another example, the effluent inlet port and the effluent outlet port may be in fluid communication with the interior lumen of the valve body when the valve is in an open position. The effluent inlet port and the effluent outlet port may be fluidly isolated from the interior lumen of the valve body when the valve is in a closed position.

Alternatively or additionally to any of the examples above, in another example, the effluent inlet port and the effluent outlet port may be in fluid communication with the interior lumen of the valve body when the annular groove of the spool is aligned with the effluent inlet port and the effluent outlet port.

Alternatively or additionally to any of the examples above, in another example, the spool valve may include a fluid inlet port and a fluid inlet tube, the fluid inlet tube extending from a first end fluidly coupled to the fluid inlet port to a second end configured to be fluidly coupled to a fluid source.

Alternatively or additionally to any of the examples above, in another example, an intermediate portion of the fluid inlet tube may be configured to be positioned within a roller pump of a thrombectomy catheter system drive unit. A flow of fluid through the fluid inlet tube may be configured to move the spool from a first closed configuration to a second open configuration. When the spool is in the second open configuration, effluent may flow freely from the thrombectomy catheter to the effluent collection bag and when the spool is in the first closed configuration, effluent flow from the thrombectomy catheter to the effluent collection bag may be precluded.

Alternatively or additionally to any of the examples above, in another example, the spool valve may include a fluid inlet port.

Alternatively or additionally to any of the examples above, in another example, the assembly may further comprise a fluid inlet tube, the fluid inlet tube extending from a first end fluidly coupled to the fluid inlet port to a second end configured to be fluidly coupled to a fluid source.

Alternatively or additionally to any of the examples above, in another example, an intermediate portion of the fluid inlet tube may be configured to be positioned within a roller pump of a thrombectomy catheter system drive unit.

Alternatively or additionally to any of the examples above, in another example, the assembly may further comprise a fluid inlet tube in fluid communication with an interior of the valve. A fluid passing through the fluid inlet tube into the interior of the valve may actuate the valve from a closed position to an open position and a flow of effluent from the thrombectomy catheter to the effluent collection bag may be permitted when the valve is in the open position and a flow of effluent from the thrombectomy catheter to the effluent collection bag may be prevented when the valve is in the closed position.

Alternatively or additionally to any of the examples above, in another example, the valve may be a spool valve including a spool movable between the open position and the closed position. The fluid passing through the fluid inlet tube into the interior of the valve may be configured to move the spool from the closed position to the open position.

Alternatively or additionally to any of the examples above, in another example, the valve may include an effluent inlet port fluidly coupled to the effluent waste tube downstream of the connection manifold assembly and an effluent outlet port fluidly coupled to the effluent waste tube upstream of the effluent collection bag. Effluent may flow freely from the thrombectomy catheter to the effluent collection bag when the valve is in an open position and effluent may be prevented from flowing from the thrombectomy catheter to the effluent collection bag when the valve is in a closed position.

Alternatively or additionally to any of the examples above, in another example, the valve may include a fluid inlet port in fluid communication with a fluid inlet tube extending from the valve. Pressurized fluid within the fluid inlet tube may actuate the valve from the closed position to the open position.

Alternatively or additionally to any of the examples above, in another example, the effluent inlet port and the effluent outlet port may extend at a generally orthogonal angle to one another.

Alternatively or additionally to any of the examples above, in another example, the assembly may further comprise one or more apertures extending from an outer surface to an inner surface of the spool.

Alternatively or additionally to any of the examples above, in another example, the assembly may further comprise a plurality of O-rings positioned between an outer surface of the spool and an inner surface of the valve body.

Alternatively or additionally to any of the examples above, in another example, when the valve is in a closed configuration at least one O-ring of the plurality of O-rings may be positioned adjacent to a first side of the effluent inlet port and a second O-ring of the plurality of O-rings may be positioned adjacent to a second side of the effluent inlet port.

Alternatively or additionally to any of the examples above, in another example, the assembly may further comprise first and second O-rings positioned between an outer surface of the spool and an inner surface of the valve body.

Alternatively or additionally to any of the examples above, in another example, when the valve is in a closed configuration the effluent inlet port is positioned between the first and second O-rings.

Alternatively or additionally to any of the examples above, in another example, the assembly may further comprise a fluid outlet port.

Alternatively or additionally to any of the examples above, in another example, the fluid outlet port may be in fluid communication with the fluid inlet port.

Alternatively or additionally to any of the examples above, in another example, the fluid outlet port may be configured to selectively allow a flow of fluid from fluid inlet port to a secondary collection bag during use of the thrombectomy catheter.

Alternatively or additionally to any of the examples above, in another example, the fluid outlet port may extend in line with a longitudinal axis to the valve body.

Alternatively or additionally to any of the examples above, in another example, the effluent outlet port may extend line with a longitudinal axis to the valve body.

In another example, a thrombectomy catheter and pump assembly may comprise a thrombectomy catheter, a pump, a connection manifold assembly positioned between the thrombectomy catheter and the pump, an effluent return tube fluidly coupled to the thrombectomy catheter and the connection manifold assembly, an effluent collection bag, an effluent waste tube fluidly coupled to the connection manifold assembly and the effluent collection bag, and a spool valve positioned in line with the effluent waste tube. The spool valve may comprise a valve body, an effluent inlet port in fluid communication with a first portion of the effluent waste tube, an effluent outlet port in fluid communication with a second portion of the effluent waste tube, a fluid inlet port, the fluid inlet port at least substantially fluidly isolated from the effluent inlet port and the effluent outlet port, and a spool movably disposed within an interior lumen of the valve body. The spool may be configured to move between a closed configuration configured to fluidly isolate the effluent inlet port and the effluent outlet port from one another and an open configuration configured to fluidly couple the effluent inlet port and the effluent outlet port.

Alternatively or additionally to any of the examples above, in another example, a flow of fluid into the fluid inlet port during operation of the thrombectomy catheter may be configured to move the spool from the closed configuration to the open configuration.

Alternatively or additionally to any of the examples above, in another example, when the spool is in the open configuration effluent may flow freely from the thrombectomy catheter to the effluent collection bag.

In another example, a thrombectomy system may comprise a drive unit, a roller pump driven by the drive unit, a fluid inflow pump, the fluid inflow pump driven by the drive unit, a thrombectomy catheter, the fluid inflow pump configured to provide fluid inflow through the thrombectomy catheter, an effluent collection bag, an effluent waste tube fluidly extending between the thrombectomy catheter and the effluent collection bag for passing an effluent from the thrombectomy catheter to the effluent collection bag, and a valve positioned in line with the effluent waste tube upstream of the effluent collection bag. The valve may comprise a valve body, an effluent inlet port, an effluent outlet port, and a fluid inlet port fluidly coupled to a fluid inlet tube, the fluid inlet port at least substantially fluidly isolated from the effluent inlet port and the effluent outlet port. A portion of the fluid inlet tube may be disposed within the roller pump. The valve may be configured to move between a closed configuration configured to fluidly isolate the effluent inlet port and the effluent outlet port from one another and an open configuration configured to fluidly couple the effluent inlet port and the effluent outlet port in response to a flow of fluid through the fluid inlet tube by activation of the roller pump.

Alternatively or additionally to any of the examples above, in another example, the valve may be a spool valve having a spool movably disposed within an interior lumen of the valve, wherein the spool is moved to the open position via pressure of the fluid in the fluid inlet tube.

The above summary of some example embodiments is not intended to describe each disclosed embodiment or every implementation of the disclosure.

### BRIEF DESCRIPTION OF THE DRAWINGS

The disclosure may be more completely understood in consideration of the following detailed description of various embodiments in connection with the accompanying drawings, in which:
FIG. 1 is a perspective view of an illustrative thrombectomy system;
FIG. 2 is a partially exploded perspective view of the pump, the bubble trap, the connection manifold assembly, and an associated fixture of the pump/catheter assembly for use in the thrombectomy system of FIG. 1;
FIG. 3 is a partially exploded side view of the pump, the bubble trap, the connection manifold assembly, and associated fixture of the pump/catheter assembly for use in the thrombectomy system of FIG. 1;
FIG. 4 illustrates components of a thrombectomy catheter assembly relative to associated components of a console of the thrombectomy system of FIG. 1;
FIG. 5 illustrates a schematic thrombectomy catheter assembly for use in the thrombectomy system of FIG. 1;
FIG. 6 is a perspective view of an illustrative spool valve for use with the illustrative thrombectomy catheter assembly;
FIG. 7 is a cross-sectional view of the illustrative spool valve of FIG. 6 in a first configuration;
FIG. 8 is a cross-sectional view of the illustrative spool valve of FIG. 6 in a second configuration;
FIG. 9 is a cross-sectional view of the illustrative spool valve of FIG. 6 in a first configuration with additional features;
FIG. 10 is a cross-sectional view of an alternative spool for use with a spool valve;
FIG. 11 is a cross-sectional view of another illustrative spool for use with a spool valve;
FIG. 12 is a cross-sectional view of an illustrative fluid inlet tube positioned in a roller pump; and
FIG. 13 is a cross-sectional view of another illustrative fluid inlet tube positioned in a roller pump;
FIG. 14 is a perspective view of another illustrative spool valve for use with the illustrative thrombectomy catheter assembly;
FIG. 15 is a cross-sectional view of the illustrative spool valve of FIG. 14 in a first configuration;
FIG. 16 is a cross-sectional view of the illustrative spool valve of FIG. 14 in a second configuration;
FIG. 17 is a perspective view of another illustrative spool valve for use with the illustrative thrombectomy catheter assembly;
FIG. 18 is a cross-sectional view of the illustrative spool valve of FIG. 17 in a first configuration;
FIG. 19 is a cross-sectional view of the illustrative spool valve of FIG. 17 in a second configuration;
FIG. 20 is a perspective view of another illustrative spool valve for use with the illustrative thrombectomy catheter assembly;
FIG. 21 is a cross-sectional view of the illustrative spool valve of FIG. 20 in a first configuration;
FIG. 22 is a cross-sectional view of the illustrative spool valve of FIG. 20 in a second configuration;
FIG. 23 is a perspective view of another illustrative spool valve for use with the illustrative thrombectomy catheter assembly;
FIG. 24 is a cross-sectional view of the illustrative spool valve of FIG. 23 in a first configuration; and
FIG. 25 is a cross-sectional view of the illustrative spool valve of FIG. 23 in a second configuration.

While the disclosure is amenable to various modifications and alternative forms, specifics thereof have been shown by way of example in the drawings and will be described in detail. It should be understood, however, that the intention is not to limit aspects of the disclosure to the particular embodiments described. On the contrary, the intention is to cover all modifications, equivalents, and alternatives falling within the scope of the disclosure.

### DETAILED DESCRIPTION

All numeric values are herein assumed to be modified by the term "about", whether or not explicitly indicated. The term "about" generally refers to a range of numbers that one of skill in the art would consider equivalent to the recited value (e.g., having the same function or result). In many instances, the term "about" may be indicative as including numbers that are rounded to the nearest significant figure.

The recitation of numerical ranges by endpoints includes all numbers within that range (e.g., 1 to 5 includes 1, 1.5, 2, 2.75, 3, 3.80, 4, and 5).

Although some suitable dimensions, ranges and/or values pertaining to various components, features and/or specifications are disclosed, one of skill in the art, incited by the present disclosure, would understand desired dimensions, ranges and/or values may deviate from those expressly disclosed.

As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise.

The following detailed description should be read with reference to the drawings in which similar elements in different drawings are numbered the same. The detailed description and the drawings, which are not necessarily to scale, depict illustrative embodiments and are not intended to limit the scope of the disclosure. The illustrative embodiments depicted are intended only as exemplary. Selected features of any illustrative embodiment may be incorporated into an additional embodiment unless clearly stated to the contrary.

Thrombectomy catheters and systems may be used to remove thrombus, plaques, lesions, clots, etc. from veins or arteries. In some thrombectomy systems, a roller pump may be used to balance fluid flow in the system. For example, a roller pump may be used to control the flow of effluent from a patient through the system while an inflow pump provides a flow of fluid into the patient. However, in some instances, a roller pump may not operate fast enough to provide the necessary fluid balance between the fluid inflow and the fluid outflow (e.g., effluent). Disclosed herein is a valve assembly that allows for effluent flow when the thrombectomy catheter is operating (e.g., when an inflow pump is operating to provide fluid inflow into the patient) and prevents effluent flow when the thrombectomy catheter is not operating (e.g., when an inflow pump is not operating such that there is no fluid inflow into the patient). Further, the disclosed valve assembly may operate without the need for control signals or programming additions/changes to the thrombectomy system. For example, the disclosed valve assembly may operate without the need for electrical control signals being sent to the valve assembly and/or electrical operation of the valve assembly.

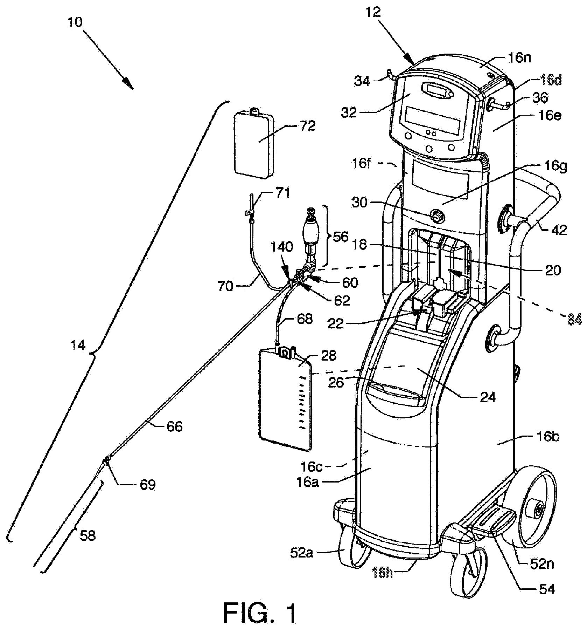

FIG. 1 is a perspective view of an illustrative thrombectomy system 10. The thrombectomy system 10 may include a control console or drive unit 12 and a pump/catheter assembly 14. In some instances, the pump/catheter assembly 14 may be a single use device in which a new pump/catheter assembly 14 may be used with the drive unit 12 for each medical procedure. Shown on the drive unit 12 are a plurality of removable panels 16a-16n about and along the drive unit 12 enclosing the internal structure of the drive unit 12. An illustrative drive unit 12 is described in commonly assigned U.S. Patent Number 7,935,077, titled THROMBECTOMY CATHETER DEPLOYMENT SYSTEM. Centrally located in the drive unit 12 and aligned to the lower region of the panel 16g may be automatically opening doors 18 and 20 which open to expose the interior of the drive unit 12 to provide access to a carriage assembly 22. The carriage assembly 22, which may accommodate components of the pump/catheter assembly 14, as discussed further herein, is shown accessible via opening the closed doors 18 and 20. The drive unit 12 may include a catch basin for collecting fluid leakage from the components of the pump/catheter assembly 14. For example, a removable drip tray 24 is shown located on the front of the drive unit 12 extending from below the carriage assembly 22 toward the panel 16a. Other configurations of catch basins are also contemplated. The drip tray 24 and a removable receptacle 26 may collectively support and accommodate an effluent collection bag, such as effluent collection bag 28 of the pump/catheter assembly 14. In other instances, the drive unit 12 may include a different structure, such as a hook for hanging the effluent collection bag 28 from, or a shelf for setting the effluent collection bag 28 on. In instances where the carriage assembly 22 is movable, a carriage assembly activation switch 30 may be provided with the drive unit 12, such as located on panel 16g to selectively position of the carriage assembly 22 inwardly or outwardly. A user interface 32, including memory capabilities, may be provided with the drive unit 12, such as located at the upper region of the drive unit 12 between the upper regions of the upper side panels 16e and 16f. Saline bag hooks 34 and 36 may extend through the panels 16e and 16f to hang saline bags therefrom. The drive unit 12 may include a handle 42 as well as a plurality of wheels 52a-52n and brake pedals 54 for wheel lockage to assist in maneuvering the drive unit 12 by medical personnel.

The pump/catheter assembly 14, which may be a disposable single-use device, is shown unattached from the drive unit 12. The pump/catheter assembly 14 includes a pump 56 and a thrombectomy catheter 58. During use, a portion of the pump/catheter assembly 14 may be secured within a portion of the drive unit 12. Other components included in the pump/catheter assembly 14 may include a bubble trap 60 attached to the pump 56, a connection manifold assembly 62 connected to the bubble trap 60, an effluent return tube 66 connected between the connection manifold assembly 62 and the thrombectomy catheter 58, a high-pressure fluid supply tube 64 attached between the output of the pump 56 and the thrombectomy catheter 58 which may be coaxially arranged inside the effluent return tube 66, a transition fixture 69 between the distal end of the effluent return tube 66 and the proximal end of the thrombectomy catheter 58, an effluent waste tube 68 connecting the effluent collection bag 28 to the connection manifold assembly 62, and a fluid supply tube 70 having a bag spike 71 connecting a fluid supply bag 72 (e.g., a saline bag) to the connection manifold assembly 62. The fluid supply tube 70 may be in fluid communication with the interior of the bubble trap 60 to provide fluid from the fluid supply bag 72 to the pump 56 and then to the thrombectomy catheter 58 through the high-pressure fluid supply tube 64.

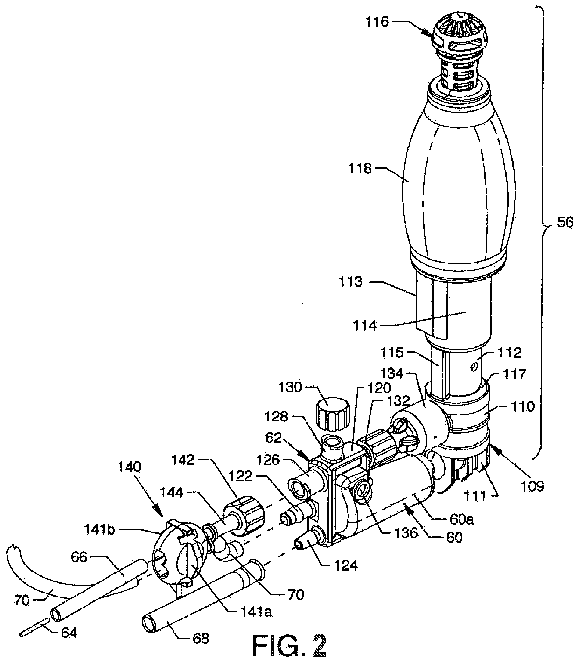

FIG. 2 is a partially exploded perspective view of several components of the pump/catheter assembly 14 generally including the pump 56, the bubble trap 60, the connection manifold assembly 62, and a fixture 140. The pump 56 centers about a tubular body 112. Components are located about the lower region of the tubular body 112 and include a base 109 having an upper portion 110 and a lower portion 111 both positioned about the lower region of the tubular body 112. An annular surface 117 is included at the top of the upper portion 110 of the base 109 for intimate contact with capture tabs of the carriage assembly 22 to contain the pump 56 within the carriage assembly 22. A top body 114, is positioned about the upper region of the tubular body 112. The base 109 and the top body 114, as well as a connecting panel 115, may be molded or otherwise suitably constructed to encompass the greater part of the tubular body 112, for example. A data plate 113 may also be included on the top body 114 for the inclusion of a barcode, an RFID tag, or other informational displays to determine operational parameters of the device.

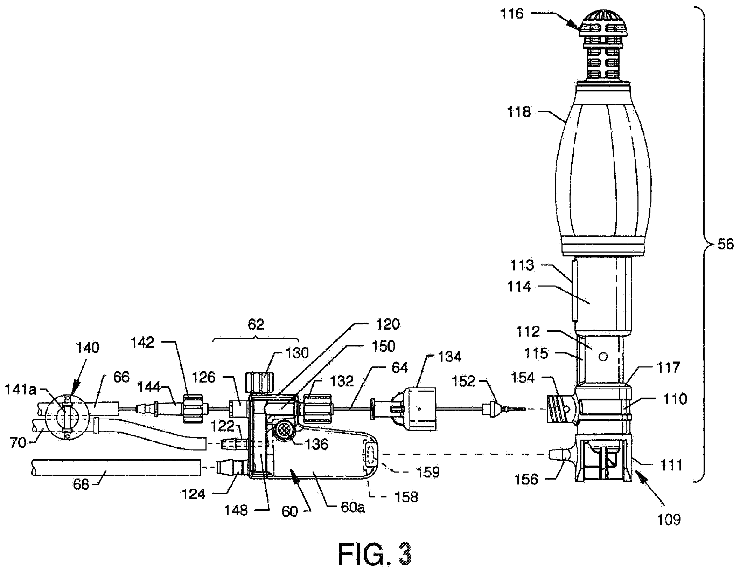

The pump 56 may include a hemispherically-shaped pump piston head 116 having a flexible boot 118 connected to and extending between the top body 114 and the pump piston head 116. In some instances, the geometrically configured lower portion 111 of the base 109 may serve as a mount for one end of the bubble trap 60 (FIG. 3).

The connection manifold assembly 62 may be secured directly to the other end of the bubble trap 60 and in some instances may include a bracket 120 to which is attached a vertically oriented tubular manifold 148 having a plurality of ports attached or formed therethrough including a fluid (e.g., saline) inlet port 122, an effluent outlet port 124, a Luer style effluent return port 126, and/or an auxiliary port 128 and cap 130. Also shown are connectors 132 and 134 connectingly extending between the connection manifold assembly 62 and the upper portion 110 of the base 109.

The bubble trap 60 may include mating halves of which one mating half 60a is shown. A hydrophobic filter 136 may be included at the upper forward region of the bubble trap half 60a. Another hydrophobic filter may be included on the second bubble trap half (not explicitly shown) which opposes the hydrophobic filter 136 on the bubble trap half 60a.

The fixture 140, and components associated therewith, assists in support and connection of the effluent return tube 66 to the effluent return port 126 by a connector 142 combined continuously with a connection tube 144, and also assists in support, passage and connection of the fluid supply tube 70 with the fluid inlet port 122. The fixture 140 may include outwardly extending vertically aligned and opposed tabs 141a and 141b which prevent the fixture 140 and associated effluent return tube 66 containing the high-pressure fluid supply tube 64 and the fluid supply tube 70 from contacting a roller pump (not explicitly shown) provided with the drive unit 12, such as located in the carriage assembly 22 or adjacent thereto.

FIG. 3 is a partially exploded side view of the elements of FIG. 2 illustrating the relationship of the pump 56, the bubble trap 60, the connection manifold assembly 62, and the fixture 140. Also shown is the vertically oriented tubular manifold 148 secured to the bracket 120. The effluent outlet port 124 may be connected to and in fluid communication with the lower interior of the tubular manifold 148. The effluent return port 126 may be connected to and in fluid communication with the upper interior of the tubular manifold 148. Also connecting to the tubular manifold 148 is a horizontally aligned passage port 150 and associated connector 132, each opposing the effluent return port 126. The passage port 150 may accommodate the high-pressure fluid supply tube 64 which extends distally through the lumen (not explicitly shown) of the passage port 150, the connector 132, the upper region of the tubular manifold 148, the effluent return port 126, the connector 142, the connection tube 144, and into and through the effluent return tube 66 in coaxial fashion to connect to the thrombectomy catheter 58 (FIG. 1). The proximal end of the high-pressure fluid supply tube 64 includes a high-pressure fitting 152 located near the proximal end of the high-pressure fluid supply tube 64 to facilitate connection of the high-pressure fluid supply tube 64 in fluid communication with the interior of the pump 56. The proximal end of the high-pressure fluid supply tube 64, which is the inlet to the high-pressure fluid supply tube 64, may include a plurality of very small holes (not shown) comprising a filter at the proximal end thereof. The connector 134, which may have internal and/or external threads, may be aligned over and about the high-pressure fluid supply tube 64 distal to the high-pressure fitting 152 and threadingly engage a threaded connection port 154 extending horizontally from the upper portion 110 of the base 109 of the pump 56. The connector 134 may be rotated to threadably engage the high-pressure fitting 152 with corresponding mating threaded structure provided with the pump 56. A connector 132 may be utilized to engage the externally threaded end of the connector 134 to secure the connector 134, and thus the pump 56, to the connection manifold assembly 62 and to provide for fixation of the bubble trap 60 to the pump 56. In addition, direct connection and fluid communication between the pump 56 and the bubble trap 60 may be provided by a horizontally oriented pump fluid inlet port 156 which engages a corresponding receptor port 158 and seal 159 interior to one end of the bubble trap 60. The fluid inlet port 122 located on the bracket 120 may extend behind the tubular manifold 148 to communicate with the interior of the bubble trap 60 for fluid (e.g., saline) debubbling, whereby unpressurized fluid (e.g., saline) is made available for use by the pump 56.

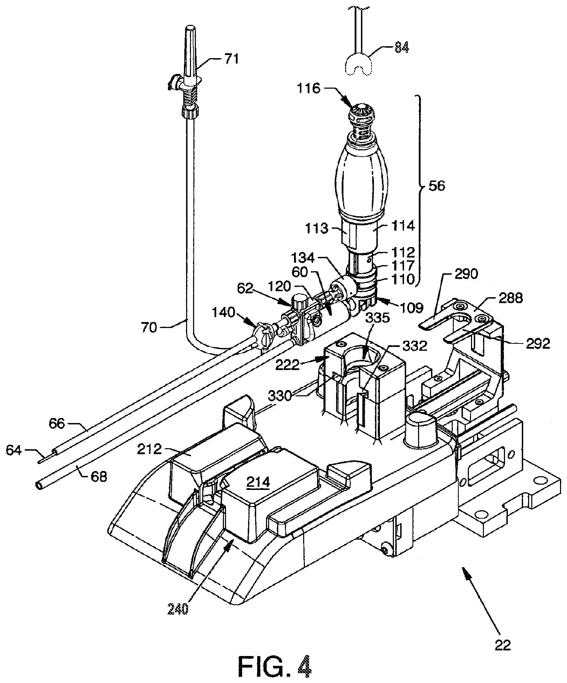

FIG. 4 is a perspective view of the illustrative disposable pump/catheter assembly 14 with the carriage assembly 22 of the drive unit 12 of the thrombectomy system 10. Generally, operation of the thrombectomy system 10 may utilize the user interface 32 for controlling the functional operation thereof in conjunction with other components. The thrombectomy system 10 may be initiated by opening a sterile package containing the disposable pump/catheter assembly 14 for loading into the drive unit 12. At a suitable time, the carriage assembly 22 may be accessed, (e.g., the doors 18, 20 may be opened on the drive unit 12) for acceptance of various components of the pump/catheter assembly 14. For example, the doors 18, 20 may be automatically opened consequent a user initiating a procedure with the user interface 32.

In some instances, the pump 56 may be aligned to the receptor slot 335 of the capture block 222 of the carriage assembly 22 and then the base 109 of the pump 56 is urged into engagement with the receptor slot 335 of the capture block 222. In embodiments in which the carriage assembly 22 is movable, a carriage motor (not explicitly shown) may then be energized, such as by depressing the carriage assembly activation switch 30 or selecting an input on the user interface 32, to movably actuate the carriage assembly to the closed position in which the pump piston head 116 is aligned with a reciprocating linear actuator 84 (shown in FIG. 4) of the drive unit 12. Capture of the pump 56 in the receptor slot 335 of the capture block 222 may occur during inwardly directed advancement of the capture block 222 when the slots 330 and 332 of the capture block 222 engage the capture tabs 290, 292 of the capture clip 288 at which time simultaneous engagement of the annular surface 117 of the pump 56 by the capture tabs 290, 292 occurs. Capturing of the pump 56 provides for secure and stable mounting and support of the pump 56 and the components directly associated with the pump 56, such as, but not limited to, the bubble trap 60, the connection manifold assembly 62 and proximal ends of the effluent waste tube 68, the saline supply tube 70, the effluent return tube 66, and other associated structure. In other embodiments in which the carriage assembly 22 is stationary, the pump piston head 116 may be aligned with the reciprocating linear actuator 84 upon engaging the base 109 of the pump 56 into the receptor slot 335. The reciprocating linear actuator 84 may then be actuated to engage and capture the pump piston head 116 such that reciprocating up and down strokes of the reciprocating linear actuator 84 drive the pump 56. Thereafter, the doors 18, 20 may be closed. The fluid supply bag 72, which may contain heparinized saline for example, may be spiked prior to or subsequent to loading the pump 56 and suitably positioned, such as on the saline bag hook 34 or 36.

The effluent waste tube 68 may also be positioned in the roller pump 240 between the tube guides 212 and 214 with the effluent collection bag 28 connected to the effluent waste tube 68. The effluent collection bag 28 may be suitably positioned for collecting effluent during the medical procedure. Pump rollers (not shown) of the roller pump 240 may rotatably engage the effluent waste tube 68 to control effluent fluid flow through the effluent waste tube 68 to the effluent collection bag 28.

At an appropriate time, the thrombectomy catheter 58 may be subjected to a priming procedure to purge the thrombectomy catheter 58 of any air. For example, the tip of the thrombectomy catheter 58 may be placed in a bowl of sterile saline, or other fluid, and the pump 56 may be operated by action of the reciprocating linear actuator 84 to prime the thrombectomy catheter 58. Thereafter, medical personnel may insert the thrombectomy catheter 58 into the vasculature of the patient at a convenient time, and operation of the thrombectomy system 10 incorporating the user interface 32 and the foot switch (not explicitly shown) can begin, as desired. The reciprocating linear actuator 84 is actuated according to the operating parameters to influence proper fluid inflow pressures, pump speed, flow rates, and the like to operate the pump 56 to deliver pressurized fluid to the thrombectomy catheter 58 via the high-pressure fluid supply tube 64 residing in the effluent return tube 66. Supply fluid is routed through the bubble trap 60 and highly pressurized by the pump 56, as previously described, and through the high-pressure fluid supply tube 64 to the thrombectomy catheter 58 for use in a thrombectomy or other related procedure. Effluent is returned through the effluent return tube 66 to the connection manifold assembly 62 for collection in the effluent collection bag 28 through the effluent waste tube 68, which may be controlled by the roller pump 240.

During some medical procedures, the flow of effluent through the effluent waste tube 68 may be controlled through the roller pump 240. For example, as the roller pump actuates (e.g., rotates), effluent may be transferred from the body of the patient through the lumen of the effluent return tube 66 to the effluent collection bag 28 through the effluent waste tube 68. The speed of the roller pump 240 may be controlled to balance fluid flow within the pump/catheter assembly 14 (e.g., balance fluid inflow into the patient's body through the fluid inflow path through the thrombectomy catheter 58 and fluid outflow from the patient's body through the fluid outflow path through the thrombectomy catheter 58 and effluent waste tube 68 into the effluent collection bag 28).

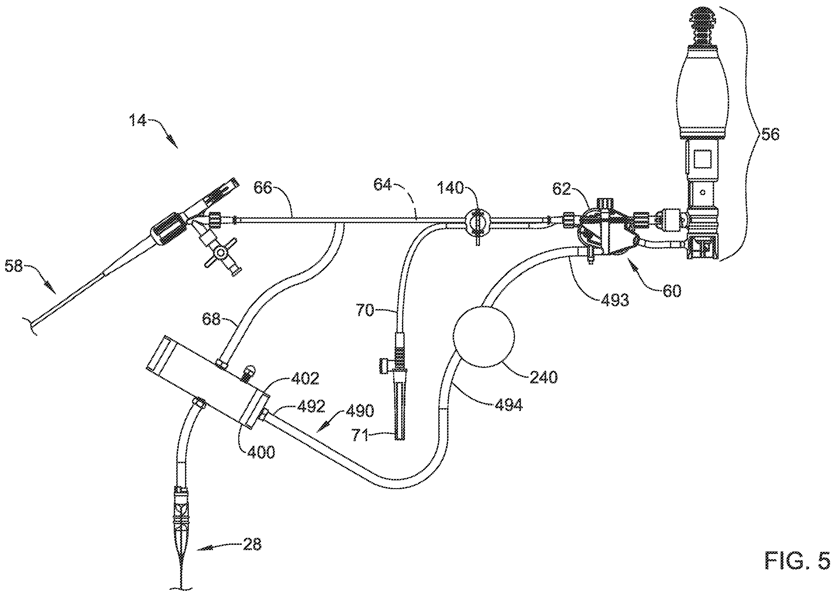

However, in some cases, the roller pump 240 may not be able to run fast enough to achieve fluid balance (e.g., the effluent outflow rate attainable by the roller pump 240 may be significantly less than the fluid inflow generated by the pump 56 such that a desired fluid balance between the fluid inflow and the effluent outflow is not able to be achieved). In such a situation, an alternative configuration of the effluent pathway may be provided, as described herein. FIG. 5 is a schematic side view of the pump/catheter assembly 14 which further includes a valve 400, such as a spool valve, to control the fluid balance in the system in instances in which a higher fluid flow rate (e.g., a higher effluent fluid flow rate) is desired during a thrombectomy procedure. Although the valve 400 is described herein as a spool valve, it is contemplated that the valve 400 may be of a different configuration, if desired. As will be described in more detail herein, the spool valve 400 is positioned in line with the effluent waste tube 68 and is configured to allow flow of effluent through the valve 400 to the waste bag 28 when the pump/catheter assembly 14 is in use (i.e., when the pump 56 is activated and running to provide fluid inflow through the thrombectomy catheter 58) and prevent flow of effluent through the valve 400 to the waste bag 28 when the pump/catheter assembly 14 is not in use (i.e., when the pump 56 is deactivated and not running). A fluid inlet tube 490 may be fluidly coupled to a first end 402 of the spool valve 400. The fluid inlet tube 490 may extend from a first end 492 coupled to the spool valve 400 to a second end493. In some instances, the second end 493 of the fluid inlet tube 490 may be open to the ambient air, or the second end 493 of the fluid inlet tube 490 may be fluidly coupled to a fluid source. In some embodiments, the fluid source may be a fluid (e.g., saline) supply bag. In some cases, the fluid bag may be the same fluid supply bag 72 that supplies saline or other fluid to the thrombectomy catheter 58. For example, a Y-connector may be utilized to allow fluid (e.g., saline) to flow from the fluid supply bag 72 to both the thrombectomy catheter 58 and the spool valve 400. In other examples, the second end 493 of the fluid inlet tube 490 may be connected to the fluid source (e.g., fluid supply bag 72) via the bubble trap 60. In yet other embodiments the fluid inlet tube 490 may be fluidly coupled to a separate fluid supply bag. In further embodiments, the fluid inlet tube 490 may be fluidly coupled to a different fluid source, such as, but not limited to, water, ambient air, etc. An intermediate portion 494 of the fluid inlet tube 490 may be positioned within the roller pump 240 in place of the effluent waste tube 68 to supply the fluid to the spool valve 400 as will be described in more detail herein. Thus, during a medical procedure in which the spool valve 400 is used with the pump/catheter assembly 14, the effluent waste tube 68 may not be positioned in the roller pump 240, but rather, the effluent waste tube 68 may branch from the effluent return tube 66 using, for example, a T-coupler or other appropriate splitting mechanism and the fluid inlet tube 490 to the spool valve 400 may be placed in the roller pump 240 such that the outflow from the roller pump 240 passes to the spool valve 400 through the fluid inlet tube to actuate the spool valve 400 to an open position. The spool valve 400 may be placed in the fluid pathway of the effluent waste tube 68 upstream of the effluent waste bag 28 such that when the spool valve 400 is in an open position the effluent passes through the spool valve 400 to the effluent waste bag 28, thus providing an outflow of effluent from the thrombectomy catheter 58, and when the spool valve 400 is in a closed position the effluent is prevented from passing through the spool valve 400 to the effluent waste bag 28, thus preventing an outflow of effluent from the thrombectomy catheter 58. Thus, the spool valve 400 may be actuated to the open position when the roller pump 240 is running and the spool valve 400 may be actuated to the closed position when the roller pump 240 is not running. Activation of the roller pump 240 may be tied to activation of the pump 56, such that when the pump 56 is running or activated (e.g., via user input to the drive unit 12), the roller pump 240 is automatically started or activated (e.g.., via a control signal from the drive unit 12). Likewise, when the pump 56 is shut off or deactivated (e.g., via user input to the drive unit 12), the roller pump 240 is automatically shut off or deactivated (e.g., via a control signal from the drive unit 12).

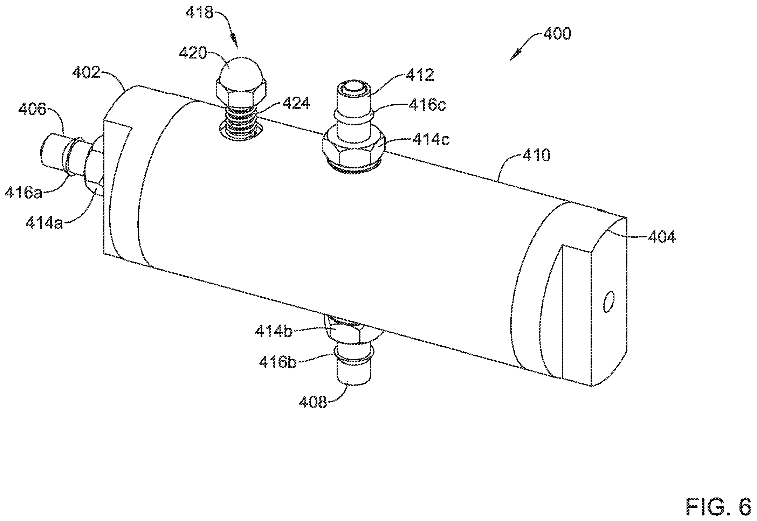

FIG. 6 is a perspective view of the illustrative spool valve 400. The spool valve 400 may include a valve body 410, which in some instances may have a generally tubular cylindrical structure, extending from a first end 402 to a second end 404. A fluid inlet port 406 may extend from the first end 402. The fluid inlet port 406 may be configured to be fluidly coupled to the fluid inlet tube 490. For example, the fluid inlet port 406 may be configured such that the fluid inlet tube 490 may be disposed over and surround the fluid inlet port 406 or may be configured such that the fluid inlet tube 490 extends within the fluid inlet port 406, as desired. In other instances, the fluid inlet tube 490 may be threadably connected to the fluid inlet port 406. The fluid inlet port 406 may be removably coupled to the valve body 410 using, for example, a removable threaded fitting 414a. Other coupling means, such as, but not limited to, press-fit, snap fit, friction fit, snap rings, etc., may be used, as desired. In some embodiments, the fluid inlet port 406 may include a tube barb including one or more raised ridges 416a. The raised ridge 416a may increase in diameter towards the valve body 410. This may facilitate assembly of the fluid inlet tube 490 with the fluid inlet port 406 while hindering accidental removal of the fluid inlet tube 490 from the fluid inlet port 406.

An effluent inlet port 408 and an effluent outlet port 412 may extend from the valve body 410, such as from the curved side wall of the valve body 410. Each of the effluent inlet port 408 and the effluent outlet port 412 may be configured to be fluidly coupled to portions of the effluent waste tube 68. A first portion of the effluent waste tube 68 may be fluidly coupled to the effluent inlet port 408 (e.g., disposed over the effluent inlet port 408 or within the effluent inlet port 408) and extend therefrom, as desired. A second portion of the effluent waste tube 68 may be fluidly coupled to the effluent outlet port 412 (e.g., disposed over the effluent outlet port 412 or within the effluent outlet port 412) and extend therefrom, as desired. The effluent inlet port 408 and the effluent outlet port 412 may be fluidly isolated from the fluid inlet port 406. In some cases, the effluent inlet port 408 and the effluent outlet port 412 may be substantially fluidly isolated from the fluid inlet port 406. For example, when the effluent inlet port 408 and the effluent outlet port 412 are substantially fluidly isolated from the fluid inlet port 406, a small pressure dissipation fluid path may extend through the spool valve 400 from the fluid inlet port 406 to the effluent inlet port 408 and/or the effluent outlet port 412 that allows pressure in the fluid inlet tube 490 to dissipate from the spool valve 400 to close the spool valve 400, as will be described in more detail herein.

In some instances, the effluent inlet port 408 and the effluent outlet port 412 may be positioned on opposite sides of the valve body 410, such as approximately 180° from one another. For example, the effluent inlet port 408 and the effluent outlet port 412 may be positioned along a common axis and extend along the common axis from opposite sides of the valve body 410. Other positions and/or configurations of the effluent inlet port 408 and the effluent outlet port 412 may be used as desired. Each of the effluent inlet port 408 and the effluent outlet port 412 may be removably coupled to the valve body 410 using, for example, a removable threaded nut 414b, 414c. Other coupling means, such as, but not limited to, press-fit, snap fit, friction fit, snap rings, etc., may be used, as desired. One or both of the effluent inlet port 408 and the effluent outlet port 412 may include a raised ridge 416b, 416c. The raised ridge 416b, 416c may increase in diameter towards the valve body 410. This may facilitate assembly of the effluent waste tube 68 with the effluent inlet port 408 and/or the effluent outlet port 412 while hindering accidental removal of the effluent waste tube 68 from the effluent inlet port 408 and/or the effluent outlet port 412. As described above, the spool valve 400 may be positioned in line with the effluent waste tube 68. For instance, the effluent inlet port 408 may be positioned downstream of the connection manifold assembly 62 and the effluent outlet port 412 may be positioned upstream of the effluent collection bag 28. For example, effluent may flow from the thrombectomy catheter 58 through the effluent return tube 66, through the connection manifold assembly 62, and into the effluent waste tube 68. Once in the effluent waste tube 68, the effluent may pass through the spool valve 400 before then entering the effluent collection bag 28.

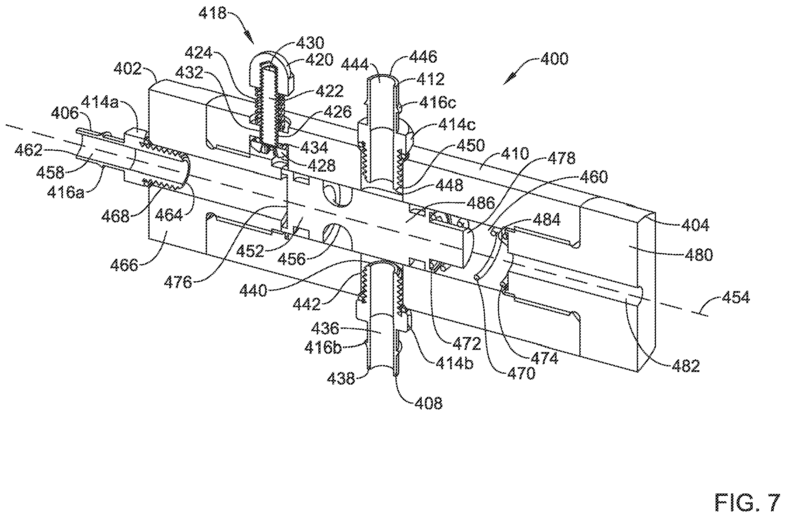

The spool valve 400 may further include a pressure relief valve or port 418. Generally, the pressure relief valve or port 418 may be actuated to prime air out of the spool valve 400 prior to use. The pressure relief valve or port 418 may include an actuation element 420, such as, but not limited to, a nut cap. Referring additionally to FIG. 7, which is a cross-sectional view of the spool valve 400 in a first or closed configuration, the actuation element 420 may be releasably coupled to a threaded region 426 of a screw or post 422. The post 422 may extend from a head portion 428 positioned within the valve body 410 to a second end 430 outside of the valve body 410. The threaded region 426 may extend through an opening or aperture 432 extending through the side wall of the valve body 410. The head portion 428 may have a diameter or cross-sectional dimension greater that the aperture 432 to prevent the post 422 from disengaging from the valve body 410. An O-ring 434 or other sealing member may be positioned between the head portion 428 and an inner surface of the side wall of the valve body 410 to fluidly seal the aperture 432. A spring 424 or other biasing mechanism may be positioned between the actuation element 420 and an outer surface of the valve body 410. The spring 424 may be positioned to surround the threaded region 426 of the post 422 and configured to bias the actuation element 420 away from an outer surface of the valve body 410 in the absence of an external force. The actuation element 420 may be pressed towards the valve body 410 to compress the spring 424 and move the O-ring 434 and the head portion 428 away from the inner surface of the side wall of the valve body 410 to allow air to exit from an interior of the valve body 410 through the aperture 432. When the force is removed from the actuation element 420, the spring 424 biases the actuation element 420 away from the outer surface of the wall of the valve body 410 and moves the O-ring 434 and head portion 428 into engagement with the aperture 432 and inner surface of the wall of the valve body 410 to fluidly seal the aperture 432.

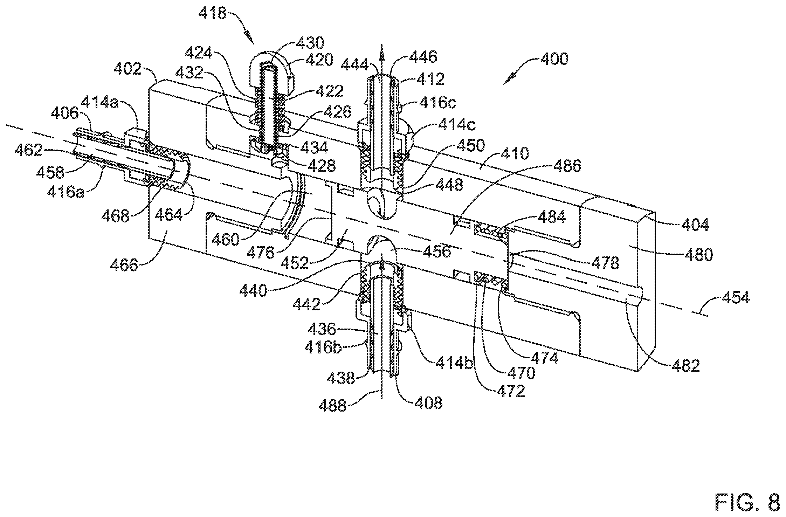

Still referring to FIG. 7 and referring additionally to FIG. 8, which is a cross-sectional view of the spool valve 400 in a second or open configuration, the effluent inlet port 408 may define a lumen 436 extending from a first end 438 external to the valve body 410 to a second end 440 disposed interior to the valve body 410. The effluent inlet port 408 may extend into a through hole 442 extending through a side wall of the valve body 410. The through hole 442 may extend through a thickness of the valve body 410 in a direction generally orthogonal to a longitudinal axis 454 of the spool valve 400 to define an opening from an exterior of the valve body 410 to an interior chamber or lumen 460 of the valve body 410. Similarly, the effluent outlet port 412 may define a lumen 444 extending from a first end 446 external to the valve body 410 to a second end 448 disposed interior to the valve body 410. The effluent outlet port 412 may extend into a through hole 450 extending through a side wall of the valve body 410. The through hole 450 may extend through a thickness of the valve body 410 in a direction generally orthogonal to the longitudinal axis 454 of the spool valve 400 to define an opening from an exterior of the valve body 410 to an interior lumen 460 of the valve body 410. The through holes 442, 450 may be in selective fluid communication with the interior lumen 460 and each other. In other instances, the effluent inlet port 408 and/or the effluent outlet port 412 may be formed integrally with the valve body 410 such that the valve body 410 is a monolithic structure including the effluent inlet port 408 and/or the effluent outlet port 412.

A generally cylindrical spool or piston 452 may be slidably disposed within the interior lumen 460 of the valve body 410. The spool 452 may be actuatable along or co-linear with the longitudinal axis 454 of the spool valve 400. In a first or closed configuration (FIG. 7), the spool 452 may be positioned to block or close off the through holes 442, 450 and hence block or close off the lumens 436, 444 of the effluent inlet port 408 and the effluent outlet port 412. In a second or open configuration (FIG. 8), the spool 452 may be displaced along the longitudinal axis 454 to align a fluid pathway through or around the spool 452 to fluidly connect the lumen 436 of the effluent inlet port 408 with the lumen 444 of the effluent outlet port 412. For example, the spool 452 may include an annulus or annular groove 456 formed about a circumference of an outer surface of the spool 452 that may be moved into alignment with the through holes 442, 450 in the second, or open configuration. This may fluidly couple the lumen 436 of the effluent inlet port 408 with the lumen 444 of the effluent outlet port 412. It is noted that in the first, or closed configuration, the annular groove 456 of the spool 452 may be moved out of alignment with the through holes 442, 450 to fluidly isolate the effluent inlet port 408 from the effluent outlet port 412.

The spool 452 may vary in diameter over a length thereof. An intermediate portion 486 of the spool 452 may have an outer diameter that is substantially the same as the inner diameter of the interior lumen 460 so as to form a fluid tight seal at the through holes 442, 450 when the spool 452 is in the closed configuration. In some embodiments, the spool 452 may be formed from a resilient material, such as, but not limited to, rubber, silicone, etc. to allow portions of the spool 452 to contact an inner surface of the valve body 410 and create a fluid tight seal. While not explicitly shown, in some cases, sealing materials, such as, but not limited to O-rings, gaskets, or other seals, etc. may be provided along an outer surface of the spool 452 or at ends thereof to provide a fluid tight seal between the spool 452 and the valve body 410. It is contemplated that the intermediate portion 486 of the spool 452 may fluidly isolate a first end region 476 of the spool 452 from a second end region 478 of the spool 452.

A spring 470 or other biasing mechanism may be disposed within the interior lumen 460 of the valve body 410, such as opposite the fluid inlet port 406. The spring 470 may extend from a first end 472 configured to be positioned adjacent to and/or in contact with a second end portion 478 of the spool 452 to a second end 474 configured to be positioned adjacent to and/or in contact with a first end 484 of a second end piece 480 of the spool valve 400. In the absence of an external force, the spring 470 may be configured to bias the spool 452 towards the first or closed configuration.

The spool 452 may be actuated to the open configuration, shown in FIG. 8, through a flow of fluid passing through the fluid inlet port 406. For example, the fluid inlet port 406 may define a lumen 458 extending from a first end 462 external to a first end piece 466 of the spool valve 400 to a second end 464 disposed interior to the first end piece 466. The fluid inlet port 406 may extend into a through hole 468 extending through the first end piece 466 along or co-linear to the longitudinal axis 454 of the spool valve 400. The through hole 468 may be in fluid communication with the interior lumen 460 of the valve body 410. Fluid may flow through the fluid inlet tube 490 and into the through hole 468 of the first end piece 466 into the interior lumen 460. Once sufficient fluid pressure has accumulated within the interior lumen 460 against the first end region 475 of the spool 452, the fluid may push the spool 452 towards the second end 404 of the spool valve 400 and compress the spring 470 to move the spool 452 from the first, closed configuration (FIG. 7) to the second, open configuration (FIG. 8). For example, sufficient fluid pressure may be the amount of pressure required to overcome the biasing force of the spring 470. As the spool 452 is actuated towards the second end 404, the second end region 478 of the spool 452 may contact the first end 484 of the second end piece 480 to provide a mechanical stop which is spaced to align the annular groove 456 with the through holes 442, 450 (and hence lumens 436, 444). In some instances, a through hole 482 may extend through the second end piece 480 to allow air to escape from the interior lumen 460 as the spool 452 is moved towards the second end 404 of the spool valve 400. Further, the through hole 482 may allow air to enter the interior lumen 460 as the spool 452 is moved towards the first end 402 of the spool valve 400.

The first and/or second end pieces 466, 480 may be provided as separate components which are assembled with the valve body 410. The first and/or second end pieces 466, 480 may be secured to the valve body 410 using any means desired such as, but not limited to, adhesives, friction fits, mechanical engagements, welding, soldering, brazing, threading, etc. In other embodiments, at least one of the first and second end pieces 466, 480 may be formed as a single monolithic structure with the valve body 410. It is contemplated that the first and/or second end pieces 466, 480 may each include a first portion configured to be received within the interior lumen 460 and a second portion configured to have an outer diameter similar to that of the valve body 410.

The spool valve 400 may be configured to open (e.g., actuate the spool 452) substantially simultaneously with the use of the thrombectomy catheter 58. For example, when the clinician activates the drive unit 12 (e.g., operates a foot switch (not explicitly shown) on the drive unit 12), the pump 56 and the roller pump 240 are both activated. In some instances, the pump 56 may be automatically activated simultaneously or sequentially with the roller pump 240 when the clinician activates the drive unit 12. As a portion 494 of the fluid inlet tube 490 is disposed within the roller pump 240 actuation of the roller pump 240 drives fluid through the fluid inlet tube 490 and into the interior lumen 460 of the valve body 410 via the fluid inlet port 406. As described above, the fluid may be saline, ambient air, or other fluid. The fluid pushes the spool 452 to the open configuration (FIG. 8) and allows for the free flow of effluent through the effluent waste tube 68, through the spool valve 400 along flow path 488 from the effluent inlet port 408 to the effluent outlet port 412, and into the effluent collection bag 28. For example, the flow of effluent is not limited by the speed of the roller pump 240. Rather, effluent may flow freely at a rate that achieves fluid flow balance within the system. As there is no fluid outlet adjacent to the first end 476 of the spool 452, the fluid flow may deadhead the roller pump 240 and no additional fluid is transferred into the interior lumen 460 of the spool valve 400, maintaining the spool 452 in the open position. When the clinician deactivates the drive unit 12 (e.g., releases the foot switch) to stop operation of the thrombectomy catheter 58), the pump 56 and the roller pump 240 are deactivated, stopping fluid inflow into the thrombectomy catheter 58. As the roller pump 240 is not a perfect pump, pressure may dissipate from the interior lumen 460 and the spring 470 may once again bias the spool 452 towards the first end 402 and the closed configuration to stop a flow of effluent through the valve 400 to the effluent waste bag 28.

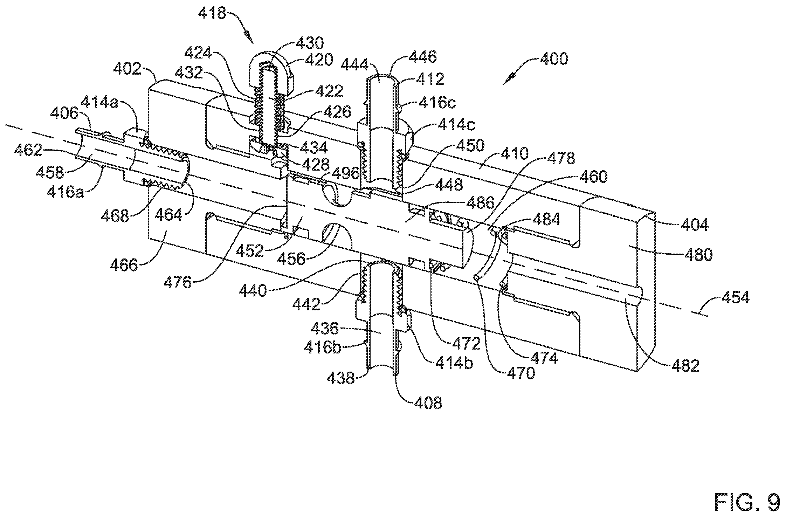

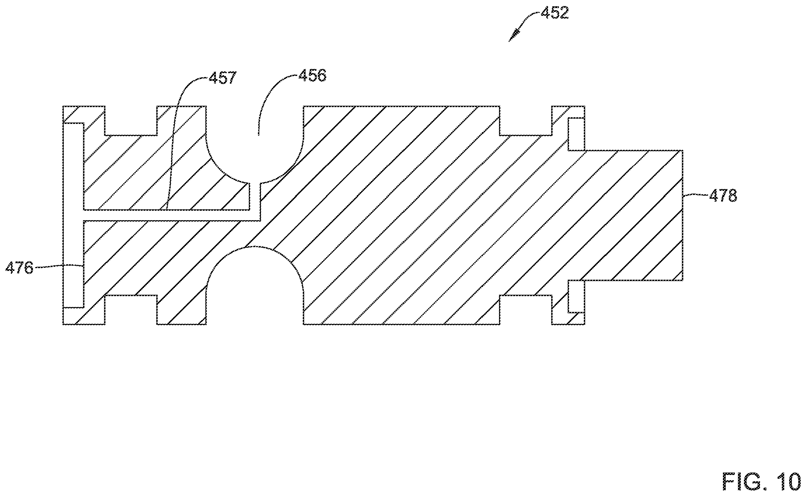

It is contemplated that other means may be provided to dissipate pressure against the spool 452 to permit the spool 452 to be returned to the closed position by the spring 470 when the drive unit 12 (including the pump 45 and the roller pump 240) is deactivated. For example, the spool valve 400 may be provided with additional features to help dissipate pressure against the spool 452 once the roller pump 240 is stopped. In some embodiments, the spool valve 400 may include a small fluid pathway (i.e., a leak path) fluidly connecting the interior lumen 460 with the effluent pathway (e.g., the lumen 444 of the effluent outlet port 412 and/or the lumen 436 of the effluent inlet port 408). The small fluid pathway may be sized such that so that a pressure head may be maintained against the spool 452 to actuate the spool 452 to the open position when the roller pump 240 is running, yet bleed off fluid in the lumen 460 to release the pressure and thus close the valve 400 (i.e., allow the spool 452 to move to the closed position) when the roller pump 240 is stopped. FIG. 9 is a cross-sectional view of the spool valve 400 in the first, closed configuration and including a small fluid pathway, shown as a longitudinally extending small leak channel e.g., groove or recess) 496 formed within the outer surface of the spool 452. The groove 496 may extend from the first end region 476 of the spool 452 towards the second end region 478 to a location on the second end side of the annular groove 456 to create a leak path that aligns with one or both of the through holes 442, 450 to further dissipate pressure when the roller pump 240 is stopped or otherwise no longer active (i.e., not rotating). The small leak channel 496, forming the leak path, may have a length sufficient to extend from the lumen 460 to the effluent pathway (e.g., the lumen 444 of the effluent outlet port 412 and/or the lumen 436 of the effluent inlet port 408) when the spool 452 is in both the open and closed positions. Other configurations of the leak path are also contemplated. For example, referring additionally to FIG. 10, which is a cross-sectional view of the illustrative spool 452, the leak path may be a small leak channel 457 extending through the interior of the spool 452 such that the small leak channel extends from the lumen 460 to the effluent pathway (e.g., the lumen 444 of the effluent outlet port 412 and/or the lumen 436 of the effluent inlet port 408) when the spool 452 is in the open position. In the illustrated embodiment, the leak channel 457 may have a generally "L" shaped configuration in which a first portion of the leak channel 457 may extend from the first end region 476 axially along the central axis of the spool 452 and a second portion may extend perpendicularly thereto (and thus perpendicular to the central axis of the spool 452 from the first portion to the annular groove 456, thus connecting the first end region 476 to the annular groove 456. However, this is not required. It is contemplated that the leak channel 457 may take other shapes, as desired. In the illustrative example, the leak channel 457 may be in fluid communication with the effluent pathway in the open position, and the leak channel 457 may be fluidly isolated from the effluent pathway in the closed position.

In another example, the leak path may be a small leak channel extending through the interior of the spool 452 such that the small leak channel extends from the lumen 460 to the effluent pathway (e.g., the lumen 444 of the effluent outlet port 412 and/or the lumen 436 of the effluent inlet port 408) when the spool 452 is in both the open and closed positions. In another embodiment the leak path may be a small leak channel extending along an interior of the valve body 410 along the spool 452 such that the small leak channel extends from the lumen 460 to the effluent pathway (e.g., the lumen 444 of the effluent outlet port 412 and/or the lumen 436 of the effluent inlet port 408) when the spool 452 is in both the open and closed positions. In yet another embodiment, the leak path may be a small leak channel extending through the valve body 410 from the lumen 460 to the exterior of the valve body 410. It is contemplated that when a leak path is provided, the pressure relief valve or port 418 may be omitted. In yet another embodiment, the leak path may be incorporated into the pressure relief valve or port 418.

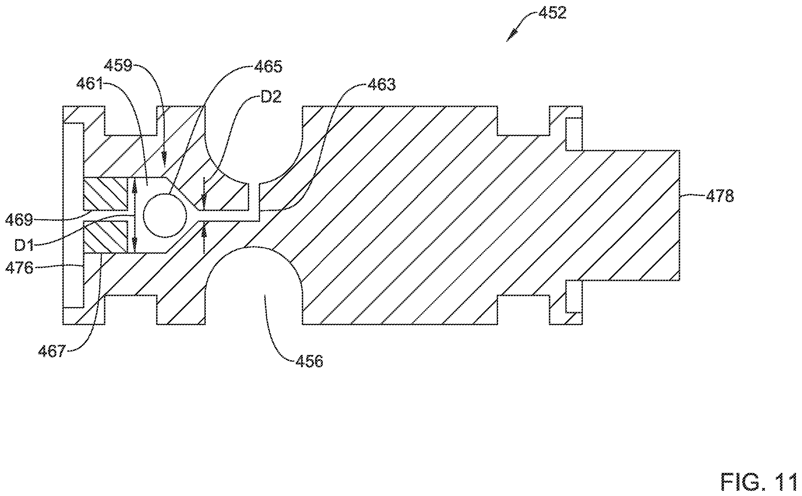

FIG. 11 is a cross-sectional view of the spool 452 including an alternative small fluid pathway 459 forming a leak channel incorporating a one-way valve in the fluid pathway 459. The fluid pathway 459 may include a first portion or channel 461. The first channel 461 may have a generally constant first diameter D1 extending from the first end region 746 of the spool 452 towards the second end region 478. The diameter D1 may transition from the first diameter to a second, smaller diameter D2 at a location between the first end region 476 and the annular groove 456. The transition may be a gradual taper or may be an abrupt stair-step transition, as desired. The first channel 461 may be in fluid communication with a second channel 463 extending from a second end of the first channel 461 to the annular groove 456. In the illustrated embodiment, the second channel 463 may have a generally "L" shaped configuration. However, this is not required. It is contemplated that the second channel 463 may take other shapes, as desired. A one-way valve, such as a check ball 465, may be positioned within the first channel 461 and a plug 467 inserted into the first channel 461 adjacent to a first end thereof. The plug 467 may be configured to close the first end of the first channel 461 to maintain the check ball 465 within the first channel 461. The plug 467 may include a leak channel 469 extending through a length thereof connecting the lumen 460 with the first channel 461 having the check ball 465 positioned therein. Collectively, the leak channel 469, the first channel 461, and the second channel 463 may create a leak path that aligns with one or both of the through holes 442, 450 to further dissipate pressure when the roller pump 240 is stopped or otherwise no longer active (i.e., not rotating).

The small fluid pathway 469, 461, 463 forming the leak pathway may be sized such that a pressure head may be maintained against the spool 452 in the lumen 460 to actuate the spool 452 to the open position when the roller pump 240 is running, yet bleed off fluid in the lumen 460 to release the pressure and thus close the valve 400 (i.e., allow the spool 452 to move to the closed position) when the roller pump 240 is stopped. For example, the roller pump 240 drives fluid through the fluid inlet tube 490 and into the interior lumen 460 of the valve body 410 via the fluid inlet port 406. The fluid pushes the spool 452 to the open configuration (FIG. 8) and allows for the free flow of effluent through the effluent waste tube 68, through the spool valve 400 along flow path 488 from the effluent inlet port 408 to the effluent outlet port 412, and into the effluent collection bag 28. Some fluid may enter the first channel 461 via the leak channel 469. The pressure of the fluid within the first channel 461 may push the check ball 465 toward the opening of the second channel 463, and thus away from the leak pathway 469 to allow a small quantity of fluid to flow around the check ball 465, into the second channel 463 and out the effluent pathway. When the roller pump 240 is deactivated, fluid inflow into the fluid inlet port 406 is stopped while some fluid remains in the lumen 460. The fluid remaining in the lumen 460 may be relieved by passing within the first channel 461 from the lumen 460 via the leak path 469 and by the check ball 465 into the second channel 463 to allow pressure to dissipate via the second channel 463, the annular groove 456, and one or both of the through holes 442, 450 in the effluent pathway. Once the pressure of the fluid in the lumen 460, and thus in the first channel 461 and the leak channel 469, has dropped below a threshold amount, the check ball 465 may move toward the leak channel 469 and seal off the leak channel 469 preventing effluent in the effluent pathway from passing by the check ball 465 into the leak channel 469 and the lumen 460. Thus, this configuration provides a one-way valve to allow fluid from the lumen 460 to pass through the one-way valve to the effluent pathway, but prevents effluent in the effluent pathway from passing through the one-way valve to the lumen 460. In some instance, a spring or other biasing member may be provided to bias the check ball 465 within the first channel 461.

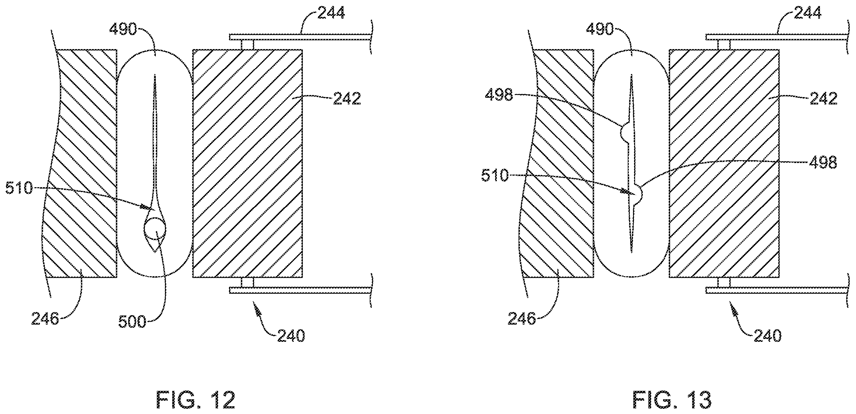

In other instances, the leak path may be provided by the portion of the fluid inlet tube 490 passing through the roller pump 240. A first example is illustrated in FIG. 12, which is a cross-sectional view illustrating a portion of the fluid inlet tube 490 being pinched together in the roller pump 240 between a roller 242 and the housing 246 of the roller pump. As generally understood, a roller pump 240 may include a rotating rotor 244 having a plurality of rollers 242 attached thereto. As the rotor 244 is rotated, the rollers 242 compress the tube 490 to push a bolus of fluid through the lumen of the tube 490 ahead of the roller 242. In the embodiment of FIG. 12, a wire 500 may be positioned in the lumen of the fluid inlet tube 490 at least throughout the portion of the fluid inlet tube 490 passing through the roller pump 240. The placement of the wire 500 within the lumen of the tube 490 may prevent the rollers 242 from completing occluding the collapsed or compressed lumen of the tube 490 between the roller 242 and the housing 246, leaving a leak channel 510 passing through the tube 490 alongside the wire 500 from a location upstream of the roller pump 240 to a location downstream of the roller pump 240. Accordingly, a small leak channel may be formed from the lumen 460 of the valve 400 through the lumen of the fluid inflow tube 490 and past the roller pump 240 such that fluid in the lumen 460 may pass in a retrograde direction through the leak channel 510 in the tube 490 when the roller pump 240 is deactivated or stopped.

Alternatively, in the embodiment of FIG. 13, a small recess 498, or a plurality of recesses 498, may be formed in the interior wall of the fluid inlet tube 490 along at least the portion of the fluid inlet tube 490 passing through the roller pump 240. The presence of the small recess 498 may prevent the rollers 242 from completing occluding the collapsed or compressed lumen of the tube 490 between the roller 242 and the housing 246, leaving a leak channel 510 passing through the tube 490 from a location upstream of the roller pump 240 to a location downstream of the roller pump 240. Accordingly, a small leak channel may be formed from the lumen 460 of the valve 400 through the lumen of the fluid inflow tube 490 and past the roller pump 240 such that fluid in the lumen 460 may pass in a retrograde direction through the leak channel 510 in the tube 490 when the roller pump 240 is deactivated or stopped.

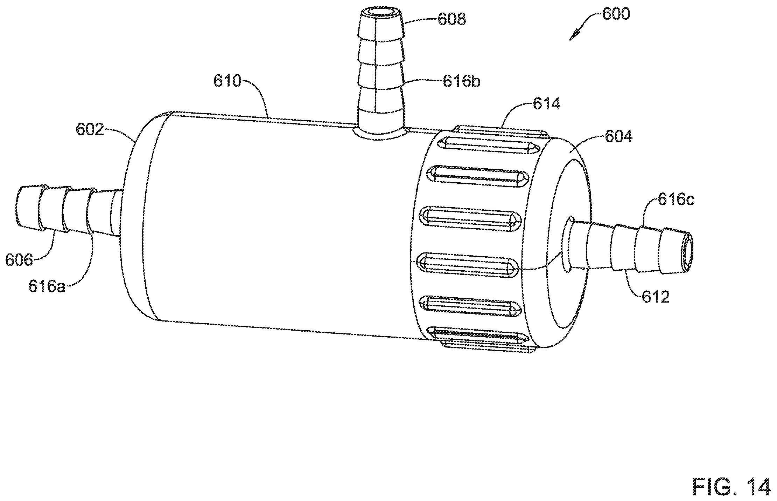

FIG. 14 is a perspective view of another illustrative spool valve 600. The spool valve 600 may include a valve body 610, which in some instances may have a generally tubular cylindrical structure, extending from a first end 602 to a second end 604. A fluid inlet port 606 may extend from the first end 602. The fluid inlet port 606 may be configured to be fluidly coupled to a fluid inlet tube, such as the fluid inlet tube 490 described with respect to FIG. 5. For example, the fluid inlet port 606 may be configured such that the fluid inlet tube 490 may be disposed over and surround the fluid inlet port 606 or may be configured such that the fluid inlet tube 490 extends within the fluid inlet port 606, as desired. In other instances, the fluid inlet tube 490 may be threadably connected to the fluid inlet port 606. The fluid inlet port 606 may be formed as a single monolithic structure with the valve body 610. In other embodiments, the fluid inlet port 606 may be removably coupled to the valve body 610 using, for example, a removable threaded fitting, press-fit, snap fit, friction fit, snap rings, etc., may be used, as desired. In some embodiments, the fluid inlet port 606 may include a tube barb including one or more raised ridges 616a. The raised ridges 616a may increase in diameter towards the valve body 610. This may facilitate assembly of the fluid inlet tube 490 with the fluid inlet port 606 while hindering accidental removal of the fluid inlet tube 490 from the fluid inlet port 606.

An effluent inlet port 608 may extend from the valve body 610, such as from the curved side wall of the valve body 610. An effluent outlet port 612 may extend from the second end 604 of the valve body 610. For example, the effluent inlet port 608 and the effluent outlet port 612 may extend at a generally orthogonal angle to one another. Each of the effluent inlet port 608 and the effluent outlet port 612 may be configured to be fluidly coupled to portions of the effluent waste tube 68. A first portion of the effluent waste tube 68 may be fluidly coupled to the effluent inlet port 608 (e.g., disposed over the effluent inlet port 608 or within the effluent inlet port 608) and extend therefrom, as desired. A second portion of the effluent waste tube 68 may be fluidly coupled to the effluent outlet port 612 (e.g., disposed over the effluent outlet port 612 or within the effluent outlet port 612) and extend therefrom, as desired. The effluent inlet port 608 and the effluent outlet port 612 may be fluidly isolated from the fluid inlet port 606. In some cases, the effluent inlet port 608 and the effluent outlet port 612 may be substantially fluidly isolated from the fluid inlet port 606. For example, when the effluent inlet port 608 and the effluent outlet port 612 are substantially fluidly isolated from the fluid inlet port 606, a small pressure dissipation fluid path may extend through the spool valve 600 from the fluid inlet port 606 to the effluent inlet port 608 and/or the effluent outlet port 612 that allows pressure in the fluid inlet tube 490 to dissipate from the spool valve 600 to close the spool valve 600, as will be described in more detail herein.

In some instances, the effluent inlet port 608 and the effluent outlet port 612 may be positioned such that their respective flow paths are approximately 90° relative to each other. Other positions and/or configurations of the effluent inlet port 608 and the effluent outlet port 612 may be used as desired. The effluent inlet port 608 may be formed as a single monolithic structure with the valve body 610. In other embodiments, the effluent inlet port 608 may be removably coupled to the valve body 610 using, for example, a removable threaded fitting, press-fit, snap fit, friction fit, snap rings, etc., may be used, as desired. In some embodiments, the effluent outlet port 612 may be removably coupled to the valve body 610 using, for example, a removable threaded valve cap 614. Other coupling means, such as, but not limited to, press-fit, snap fit, friction fit, snap rings, etc., may be used, as desired. In yet other examples, the effluent outlet port 612 may be formed as a single monolithic structure with the valve body 610. One or both of the effluent inlet port 608 and the effluent outlet port 612 may include one or more raised ridges 616b, 616c. The raised ridges 616b, 616c may increase in diameter towards the valve body 610. This may facilitate assembly of the effluent waste tube 68 with the effluent inlet port 608 and/or the effluent outlet port 612 while hindering accidental removal of the effluent waste tube 68 from the effluent inlet port 608 and/or the effluent outlet port 612.

As described above, the spool valve 600 may be positioned in line with the effluent waste tube 68. For instance, the effluent inlet port 608 may be positioned downstream of the connection manifold assembly 62 and the effluent outlet port 612 may be positioned upstream of the effluent collection bag 28. For example, effluent may flow from the thrombectomy catheter 58 through the effluent return tube 66, through the connection manifold assembly 62, and into the effluent waste tube 68. Once in the effluent waste tube 68, the effluent may pass through the spool valve 600 before then entering the effluent collection bag 28. It is contemplated that positioning the effluent outlet port 612 at a non-parallel angle to the effluent inlet port 608 or in line with the fluid inlet port 606 may allow the spool valve 600 to be hung with its longitudinal axis extending in a generally vertical direction. This in turn may allow the fluid inlet port 606 to align with the fluid inlet tube 490. It is further contemplated that placing the effluent outlet port 612 in line with the fluid inlet port 606 may allow the waste bag 28 to be easily placed on the console's drip tray 24.

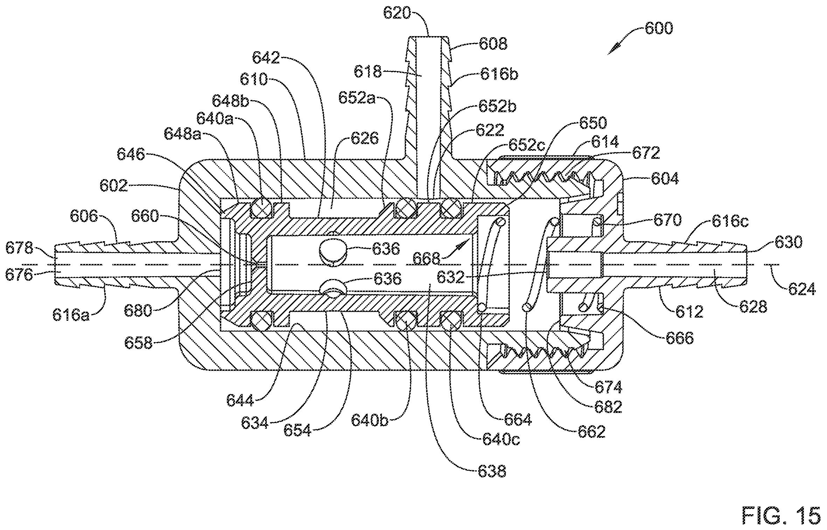

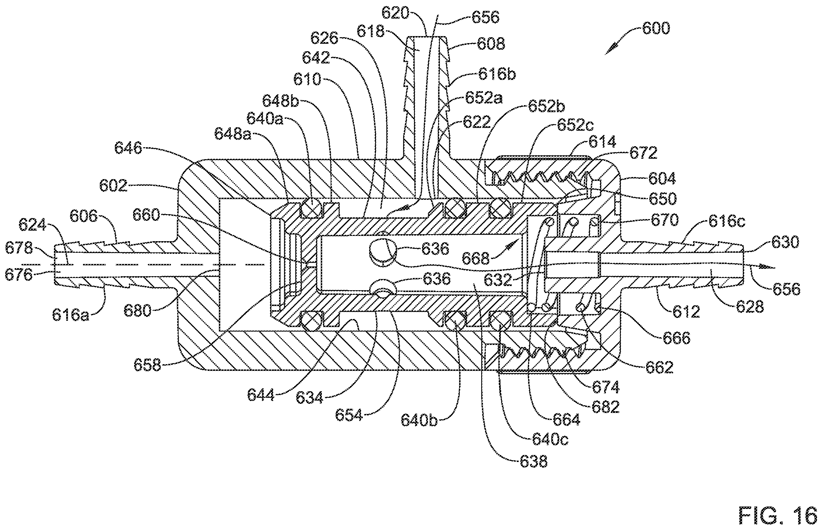

Referring additionally to FIG. 15, which is a cross-sectional view of the spool valve 600 in a first or closed configuration, and referring to FIG. 16, which is a cross-sectional view of the spool valve 600 in a second or open configuration, the effluent inlet port 608 may define a lumen 618 extending from a first end 620 external to the valve body 610 to a second end 622 disposed interior to the valve body 610. The lumen 618 of the effluent inlet port 608 may extend through a side wall of the valve body 610 in a direction generally orthogonal to a longitudinal axis 624 of the spool valve 600 to define an opening from an exterior of the valve body 610 to an interior chamber or lumen 626 of the valve body 610. The lumen 618 of the effluent inlet port 608 may be in selective fluid communication with the lumen 626 of the valve body 610. Similarly, the effluent outlet port 612 may define a lumen 628 extending from a first end 630 external to the valve body 610 to a second end 632 disposed interior to the valve body 610. The lumen 628 of the effluent outlet port 612 may extend through the valve cap 614 in a direction generally parallel to or co-linear to the longitudinal axis 624 of the spool valve 600 to define an opening from an exterior of the valve body 610 to an interior chamber or lumen 626 of the valve body 610.

A generally cylindrical spool or piston 634 may be slidably disposed within the interior lumen 626 of the valve body 610. The spool 634 may be actuatable along or co-linear with the longitudinal axis 624 of the spool valve 600. In a first or closed configuration (FIG. 15), the spool 634 may be positioned to block or close off the lumen 618 of the effluent inlet port 608. This may prevent effluent from entering the lumen 626 of the valve body 610. In a second or open configuration (FIG. 16), the spool 634 may be displaced along the longitudinal axis 624 to align a fluid pathway through the spool 634 with the lumen 618 to fluidly connect the lumen 618 of the effluent inlet port 608 with the lumen 628 of the effluent outlet port 612.

The spool 634 may vary in diameter over a length thereof. For example, the spool 634 may include an intermediate region 654, or annular groove, having a first outer diameter, longitudinally spaced raised regions 648a, 648b (collectively, 648) adjacent a first end 646 of the spool 634, and longitudinally spaced raised regions 652a, 652b (collectively, 652) adjacent a second end 650 of the spool 634. Each of the intermediate region 654 and the raised regions 648, 652 may extend about a circumference of the spool 634. The raised regions 648, 652 may have an outer diameter that is greater that the first outer diameter of the intermediate region 654. In some examples, the raised regions 648, 652 may have an outer diameter that is substantially the same as the inner diameter of the interior lumen 626 so as to form a fluid tight seal at the lumen 618 of the effluent inlet port 608 when the spool 634 is in the closed configuration. However, in other embodiments, the raised regions 648, 652 may have an outer diameter that is less than the inner diameter of the interior lumen 626. In some embodiments, the spool 634 may be formed from a resilient material, such as, but not limited to, rubber, silicone, etc. to allow portions of the spool 634 to contact an inner surface of the valve body 610 and create a fluid tight seal.

The spool 634 may include a plurality of O-rings or other sealing members 640a, 640b, 640c (collectively, 640) positioned at intervals along a length of the spool 634. The O-rings 640 may be positioned between an outer surface 642 of the spool 634 and an inner surface 644 of the valve body 610 to provide a fluid tight seal between the spool 634 and the valve body 610. A first O-ring 640a may be positioned adjacent to the first end 646 of the spool 634. The first O-ring 640a may be positioned within a recess defined by a pair of raised regions 648a, 648b. The first O-ring 640a may be configured to substantially fluidly isolate the fluid inlet port 606 from the effluent inlet port 608 and the effluent outlet port 612. A second O-ring 640b and a third O-ring 640c may be positioned adjacent to the second end 650 of the spool 634. The second and third O-rings 640b, 640c may be positioned within recesses defined by a plurality of raised regions 652a, 652b, 652c. When the spool valve 600 is in the closed configuration (FIG. 15), the second and third O-rings 640b, 640c may be positioned on a first side and a second side, respectively, of the second end 622 of the lumen 618 of the effluent inlet port 608 such that the lumen 618 of the effluent inlet port 608 is positioned between the second and third O-rings 640b, 640c. The second and third O-rings 640b, 640c may fluidly isolate the lumen 618 of the effluent inlet port 608 from the lumen 626 of the valve body 610 when the valve 600 is in the closed configuration. For example, when the valve 600 is in the closed configuration, fluid may not exit the lumen 618 of the effluent inlet port 608.

The intermediate region 654 of the spool 634 may have an outer diameter less than the diameter of the raised regions 648, 652 that may be moved into alignment with the lumen 618 of the effluent inlet port 608 in the second, or open configuration (FIG. 16). It is noted that in the first, or closed configuration, the intermediate region 654 of the spool 634 may be moved out of alignment with the lumen 618 of the effluent inlet port 608 to fluidly isolate the effluent inlet port 608 from the effluent outlet port 612 via the second and third O-rings 640b, 640c. Movement of the spool 634 to align the intermediate region 654 with the lumen 618 of the effluent inlet port 608 may fluidly couple the lumen 618 of the effluent inlet port 608 with the lumen 628 of the effluent outlet port 612. For example, the spool 634 may include one or more holes or apertures 636 extending from an outer surface of the spool 634 to an interior lumen 638 of the spool 634. The apertures 636 may be circumferentially and/or axially spaced about the intermediate region 654, as desired. As fluid passes through the lumen 618 of the effluent inlet port 608, the fluid may enter the lumen 626 of the valve body 610. The fluid may then pass through the one or more apertures 636 of the spool 634 and into an interior lumen 638 of the spool 634. The interior lumen 638 of the spool 634 may be in fluid communication with the lumen 628 of the effluent outlet port 612 to allow fluid to exit the valve body 610, as shown at flow path 656.

It is contemplated that a radially inwardly extending wall 658 of the spool 634 may extend across the interior lumen 638 thereof to substantially fluidly isolate the first end 646 of the spool 634 from the second end of the spool 650. A small aperture 660 may extend through a thickness of the radially inwardly extending wall 658 to allow a small amount of fluid received at the fluid inlet port 606 to pass into the interior lumen 638 of the spool 634. This may allow pressure to dissipate when the roller pump 240 is turned off and the valve to move from the open to the closed configuration, as will be described in more detail herein.

A spring 662 or other biasing mechanism may be disposed within the interior lumen 626 of the valve body 610, such as opposite the fluid inlet port 606. The spring 662 may extend from a first end 664 configured to be positioned adjacent to and/or in contact with a second end portion 668 of the spool 634 to a second end 666 configured to be positioned adjacent to and/or in contact with an interior surface 670 of the valve cap 614 of the spool valve 600. In the absence of an external force, the spring 662 may be configured to bias the spool 634 towards the first or closed configuration. The valve cap 614 may be configured to threadably engage the valve body 610 via a set of mating threads 672, 674. The valve cap 614 may be provided as a separate component which is assembled with the valve body 610. While the valve cap 614 is shown and described has having a threaded coupling 672, 674 with the valve body 610, the valve cap 614 may be secured to the valve body 610 using any means desired such as, but not limited to, adhesives, friction fits, mechanical engagements, welding, soldering, brazing, threading, etc. The valve cap 614 may have an outer diameter that is similar to the outer diameter of the first end region of the valve body 610. The valve cap 614 may include internal threading 672 configured to engage outer threading 674 formed on an outer surface of the valve body 610. In other embodiments, at least one of the fluid inlet port 606 and/or effluent inlet port 608 may be formed as separate components or coupled to separate components that are subsequently assembled with the valve body 610 in a manner similar to the valve cap 614.

The spool 634 may be actuated to the open configuration, shown in FIG. 16, through a flow of fluid passing through the fluid inlet port 606. For example, the fluid inlet port 606 may define a lumen 676 extending from a first end 678 external to the valve body to a second end 680 disposed interior to the valve body 610. The fluid inlet port 606 may extend along or co-linear to the longitudinal axis 624 of the spool valve 600. The lumen 676 may be in fluid communication with the interior lumen 626 of the valve body 610. Fluid may flow through the fluid inlet tube 490 and into the lumen 676 of the fluid inlet port 606 into the interior lumen 626. Once sufficient fluid pressure has accumulated within the interior lumen 626 against a first side of the radially inwardly extending wall 658 of the spool 634, the fluid may push the spool 634 towards the second end 604 of the spool valve 600 and compress the spring 662 to move the spool 634 from the first, closed configuration (FIG. 15) to the second, open configuration (FIG. 16). For example, sufficient fluid pressure may be the amount of pressure required to overcome the biasing force of the spring 662. As the spool 634 is actuated towards the second end 604, the second end 650 of the spool 634 may contact an annular wall 682 of the valve cap 614 to provide a mechanical stop which is spaced to align the intermediate region 654 with the lumen 618 of the effluent inlet port 608. In some instances, the lumen 628 of the effluent outlet port 612 may allow air to escape from the interior lumen 626 as the spool 634 is moved towards the second end 604 of the spool valve 600. Further, the lumen 628 of the effluent outlet port 612 may allow air to enter the interior lumen 626 as the spool 634 is moved towards the first end 602 of the spool valve 600.

The spool valve 600 may be configured to open (e.g., actuate the spool 634) substantially simultaneously with the use of the thrombectomy catheter 58. For example, when the clinician activates the drive unit 12 (e.g., operates a foot switch (not explicitly shown) on the drive unit 12), the pump 56 and the roller pump 240 are both activated. In some instances, the pump 56 may be automatically activated simultaneously or sequentially with the roller pump 240 when the clinician activates the drive unit 12. As a portion 494 of the fluid inlet tube 490 is disposed within the roller pump 240, actuation of the roller pump 240 drives fluid through the fluid inlet tube 490 and into the interior lumen 626 of the valve body 610 via the fluid inlet port 606. As described above, the fluid may be saline, ambient air, or other fluid. The fluid pushes the spool 634 to the open configuration (FIG. 16) and allows for the free flow of effluent through the effluent waste tube 68, through the spool valve 600 along flow path 656 from the effluent inlet port 608 to the effluent outlet port 612, and into the effluent collection bag 28. For example, the flow of effluent is not limited by the speed of the roller pump 240. Rather, effluent may flow freely at a rate that achieves fluid flow balance within the system. As there is no fluid outlet adjacent to the first end 646 of the spool 634, the fluid flow may deadhead the roller pump 240 and no additional fluid is transferred into the interior lumen 626 of the spool valve 600, maintaining the spool 634 in the open position. However, the aperture 660 in the radially inwardly extending wall 658 may allow a small amount of fluid to pass from the first side of the radially inwardly extending wall 658 and into the interior lumen 638 of the spool 634. When the clinician deactivates the drive unit 12 (e.g., releases the foot switch) to stop operation of the thrombectomy catheter 58), the pump 56 and the roller pump 240 are deactivated, stopping fluid inflow into the thrombectomy catheter 58. As the roller pump 240 is not a perfect pump, pressure may dissipate from the interior lumen 626 and the spring 662 may once again bias the spool 634 towards the first end 602 and the closed configuration to stop a flow of effluent through the valve 600 to the effluent waste bag 28. It is further contemplated that the aperture 660, defining a leak channel, in the radially inwardly extending wall 658 may allow a small amount of fluid to pass from the first side of the radially inwardly extending wall 658 and into the interior lumen 638 of the spool 634 after the roller pump 240 is deactivated to dissipate pressure from the interior lumen 626. The aperture 660 may be sized such that so that a pressure head may be maintained against the spool 634 to actuate the spool 634 to the open position when the roller pump 240 is running, yet bleed off fluid in the lumen 638 to release the pressure and thus close the valve 600 (i.e., allow the spool 634 to move to the closed position) when the roller pump 240 is stopped. For example, the aperture 660 may have a diameter in the range of about 0.1 millimeters to about 0.2 millimeters. It is further contemplated that the spool valve 600 may include any of the pressure dissipation features or structures described herein.

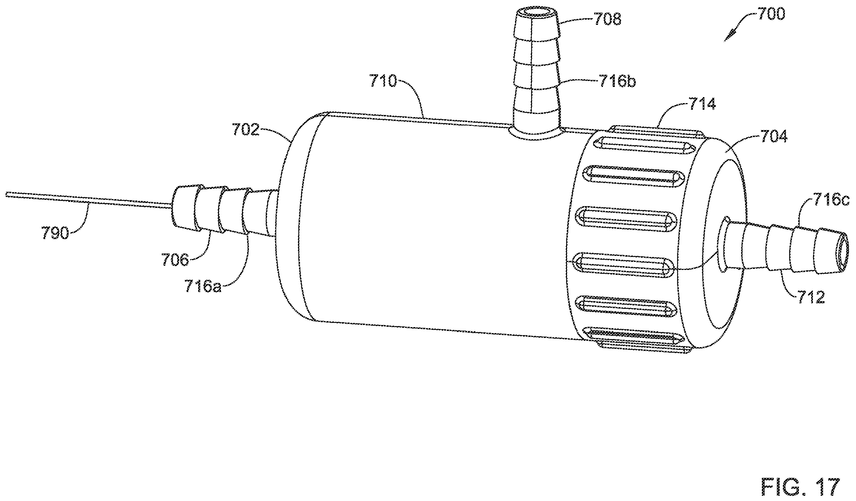

FIG. 17 is a perspective view of another illustrative spool valve 700. Generally, the spool valve 700 may be similar in form and function to the spool valve 600 described with respect to FIGS. 16-18. However, the spool valve 700 may include an alternative pressure dissipation mechanism. The spool valve 700 may include a valve body 710, which in some instances may have a generally tubular cylindrical structure, extending from a first end 702 to a second end 704. A fluid inlet port 706 may extend from the first end 702. The fluid inlet port 706 may be configured to be fluidly coupled to a fluid inlet tube, such as the fluid inlet tube 490 described with respect to FIG. 5. For example, the fluid inlet port 706 may be configured such that the fluid inlet tube 490 may be disposed over and surround the fluid inlet port 706 or may be configured such that the fluid inlet tube 490 extends within the fluid inlet port 706, as desired. In other instances, the fluid inlet tube 490 may be threadably connected to the fluid inlet port 706. The fluid inlet port 706 may be formed as a single monolithic structure with the valve body 710. In other embodiments, the fluid inlet port 706 may be removably coupled to the valve body 710 using, for example, a removable threaded fitting, press-fit, snap fit, friction fit, snap rings, etc., may be used, as desired. In some embodiments, the fluid inlet port 706 may include a tube barb including one or more raised ridges 716a. The raised ridges 716a may increase in diameter towards the valve body 710. This may facilitate assembly of the fluid inlet tube 490 with the fluid inlet port 706 while hindering accidental removal of the fluid inlet tube 490 from the fluid inlet port 706.

An effluent inlet port 708 may extend from the valve body 710, such as from the curved side wall of the valve body 710. An effluent outlet port 712 may extend from the second end 704 of the valve body 710. For example, the effluent inlet port 708 and the effluent outlet port 712 may extend at a generally orthogonal angle to one another. Each of the effluent inlet port 708 and the effluent outlet port 712 may be configured to be fluidly coupled to portions of the effluent waste tube 68. A first portion of the effluent waste tube 68 may be fluidly coupled to the effluent inlet port 708 (e.g., disposed over the effluent inlet port 708 or within the effluent inlet port 708) and extend therefrom, as desired. A second portion of the effluent waste tube 68 may be fluidly coupled to the effluent outlet port 712 (e.g., disposed over the effluent outlet port 712 or within the effluent outlet port 712) and extend therefrom, as desired. The effluent inlet port 708 and the effluent outlet port 712 may be fluidly isolated from the fluid inlet port 706. In some cases, the effluent inlet port 708 and the effluent outlet port 712 may be substantially fluidly isolated from the fluid inlet port 706. For example, when the effluent inlet port 708 and the effluent outlet port 712 are substantially fluidly isolated from the fluid inlet port 706, a small pressure dissipation fluid path may extend through the spool valve 700 from the fluid inlet port 706 to the effluent inlet port 708 and/or the effluent outlet port 712 that allows pressure in the fluid inlet tube 490 to dissipate from the spool valve 700 when the roller pump 240 is turned off to close the spool valve 700, although this is not required.

In some instances, the effluent inlet port 708 and the effluent outlet port 712 may be positioned such that their respective flow paths are approximately 90° relative to each other. Other positions and/or configurations of the effluent inlet port 708 and the effluent outlet port 712 may be used as desired. The effluent inlet port 708 may be formed as a single monolithic structure with the valve body 710. In other embodiments, the effluent inlet port 708 may be removably coupled to the valve body 710 using, for example, a removable threaded fitting, press-fit, snap fit, friction fit, snap rings, etc., may be used, as desired. In some embodiments, the effluent outlet port 712 may be removably coupled to the valve body 710 using, for example, a removable threaded valve cap 714. Other coupling means, such as, but not limited to, press-fit, snap fit, friction fit, snap rings, etc., may be used, as desired. In yet other examples, the effluent outlet port 712 may be formed as a single monolithic structure with the valve body 710. One or both of the effluent inlet port 708 and the effluent outlet port 712 may include one or more raised ridges 716b, 716c. The raised ridges 716b, 716c may increase in diameter towards the valve body 710. This may facilitate assembly of the effluent waste tube 68 with the effluent inlet port 708 and/or the effluent outlet port 712 while hindering accidental removal of the effluent waste tube 68 from the effluent inlet port 708 and/or the effluent outlet port 712.

As described above, the spool valve 700 may be positioned in line with the effluent waste tube 68. For instance, the effluent inlet port 708 may be positioned downstream of the connection manifold assembly 62 and the effluent outlet port 712 may be positioned upstream of the effluent collection bag 28. For example, effluent may flow from the thrombectomy catheter 58 through the effluent return tube 66, through the connection manifold assembly 62, and into the effluent waste tube 68. Once in the effluent waste tube 68, the effluent may pass through the spool valve 700 before then entering the effluent collection bag 28. It is contemplated that positioning the effluent outlet port 712 at a non-parallel angle to the effluent inlet port 708 or in line with the fluid inlet port 706 may allow the spool valve 700 to be hung with its longitudinal axis extending in a generally vertical direction. This in turn may allow the fluid inlet port 706 to align with the fluid inlet tube 490. It is further contemplated that placing the effluent outlet port 712 in line with the fluid inlet port 706 may allow the waste bag 28 to be easily placed on the console's drip tray 24.

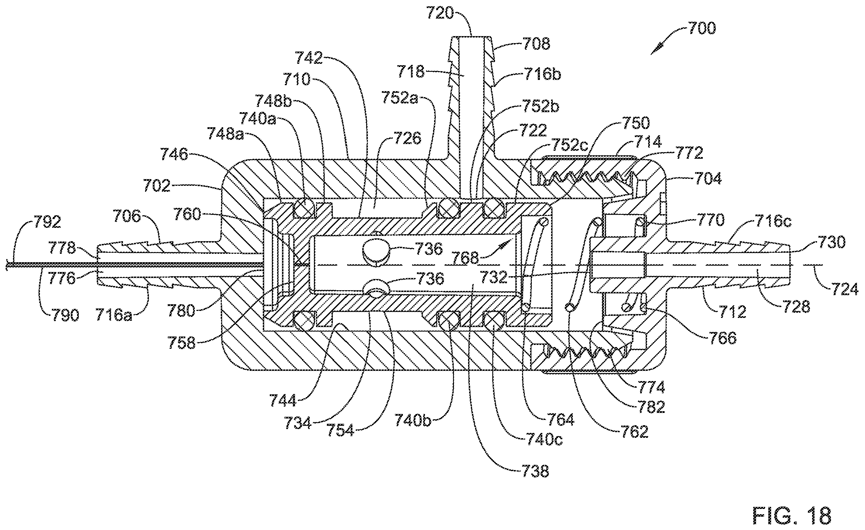

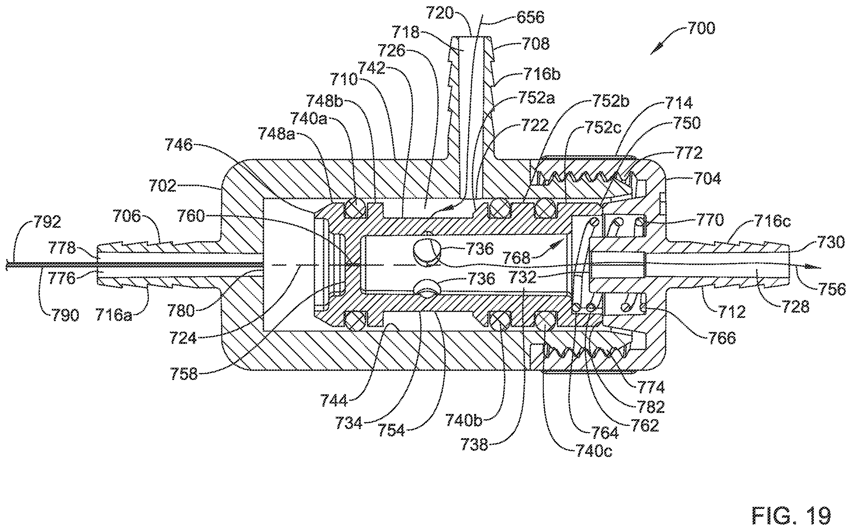

Referring additionally to FIG. 18, which is a cross-sectional view of the spool valve 700 in a first or closed configuration, and referring to FIG. 19, which is a cross-sectional view of the spool valve 700 in a second or open configuration, the effluent inlet port 708 may define a lumen 718 extending from a first end 720 external to the valve body 710 to a second end 722 disposed interior to the valve body 710. The lumen 718 of the effluent inlet port 708 may extend through a side wall of the valve body 710 in a direction generally orthogonal to a longitudinal axis 724 of the spool valve 700 to define an opening from an exterior of the valve body 710 to an interior chamber or lumen 726 of the valve body 710. The lumen 718 of the effluent inlet port 708 may be in selective fluid communication with the lumen 726 of the valve body 710. Similarly, the effluent outlet port 712 may define a lumen 728 extending from a first end 730 external to the valve body 710 to a second end 732 disposed interior to the valve body 710. The lumen 728 of the effluent outlet port 712 may extend through the valve cap 714 in a direction generally parallel to or co-linear to the longitudinal axis 724 of the spool valve 700 to define an opening from an exterior of the valve body 710 to an interior chamber or lumen 726 of the valve body 710.

A generally cylindrical spool or piston 734 may be slidably disposed within the interior lumen 726 of the valve body 710. The spool 734 may be actuatable along or co-linear with the longitudinal axis 724 of the spool valve 700. In a first or closed configuration (FIG. 18), the spool 734 may be positioned to block or close off the lumen 718 of the effluent inlet port 708. This may prevent effluent from entering the lumen 726 of the valve body 710. In a second or open configuration (FIG. 19), the spool 734 may be displaced along the longitudinal axis 724 to align a fluid pathway through the spool 734 with the lumen 718 to fluidly connect the lumen 718 of the effluent inlet port 708 with the lumen 728 of the effluent outlet port 712.

The spool 734 may vary in diameter over a length thereof. For example, the spool 734 may include an intermediate region 754, or annular groove, having a first outer diameter, longitudinally spaced raised regions 748a, 748b (collectively, 748) adjacent a first end 746 of the spool 734, and longitudinally spaced raised regions 752a, 752b (collectively, 752) adjacent a second end 750 of the spool 734. Each of the intermediate region 754 and the raised regions 748, 752 may extend about a circumference of the spool 734. The raised regions 748, 752 may have an outer diameter that is greater that the first outer diameter of the intermediate region 754. In some examples, the raised regions 748, 752 may have an outer diameter that is substantially the same as the inner diameter of the interior lumen 726 so as to form a fluid tight seal at the lumen 718 of the effluent inlet port 708 when the spool 734 is in the closed configuration. However, in other embodiments, the raised regions 748, 752 may have an outer diameter that is less than the inner diameter of the interior lumen 726. In some embodiments, the spool 734 may be formed from a resilient material, such as, but not limited to, rubber, silicone, etc. to allow portions of the spool 734 to contact an inner surface of the valve body 710 and create a fluid tight seal.

The spool 734 may include a plurality of O-rings or other sealing members 740a, 740b, 740c (collectively, 740) positioned at intervals along a length of the spool 734. The O-rings 740 may be positioned between an outer surface 742 of the spool 734 and an inner surface 744 of the valve body 710 to provide a fluid tight seal between the spool 734 and the valve body 710. A first O-ring 740a may be positioned adjacent to the first end 746 of the spool 734. The first O-ring 740a may be positioned within a recess defined by a pair of raised regions 748a, 748b. The first O-ring 740a may be configured to substantially fluidly isolate the fluid inlet port 706 from the effluent inlet port 708 and the effluent outlet port 712. A second O-ring 740b and a third O-ring 740c may be positioned adjacent to the second end 750 of the spool 734. The second and third O-rings 740b, 740c may be positioned within recesses defined by a plurality of raised regions 752a, 752b, 752c. When the spool valve 700 is in the closed configuration (FIG. 18), the second and third O-rings 740b, 740c may be positioned on a first side and a second side, respectively, of the second end 722 of the lumen 718 of the effluent inlet port 708 such that the lumen 718 of the effluent inlet port 708 is positioned between the second and third O-rings 740b, 740c. The second and third O-rings 740b, 740c may fluidly isolate the lumen 718 of the effluent inlet port 708 from the lumen 726 of the valve body 710 when the valve 700 is in the closed configuration. For example, when the valve 700 is in the closed configuration, fluid may not exit the lumen 718 of the effluent inlet port 708.

The intermediate region 754 of the spool 734 may have an outer diameter less than the diameter of the raised regions 748, 752 that may be moved into alignment with the lumen 718 of the effluent inlet port 708 in the second, or open configuration (FIG. 19). It is noted that in the first, or closed configuration, the intermediate region 754 of the spool 734 may be moved out of alignment with the lumen 718 of the effluent inlet port 708 to fluidly isolate the effluent inlet port 708 from the effluent outlet port 712 via the second and third O-rings 740b, 740c. Movement of the spool 734 to align the intermediate region 754 with the lumen 718 of the effluent inlet port 708 may fluidly couple the lumen 718 of the effluent inlet port 708 with the lumen 728 of the effluent outlet port 712. For example, the spool 734 may include one or more holes or apertures 736 extending from an outer surface of the spool 734 to an interior lumen 738 of the spool 734. The apertures 736 may be circumferentially and/or axially spaced about the intermediate region 754, as desired. As fluid passes through the lumen 718 of the effluent inlet port 708, the fluid may enter the lumen 726 of the valve body 710. The fluid may then pass through the one or more apertures 736 of the spool 734 and into an interior lumen 738 of the spool 734. The interior lumen 738 of the spool 734 may be in fluid communication with the lumen 728 of the effluent outlet port 712 to allow fluid to exit the valve body 710, as shown at flow path 756.

It is contemplated that a radially inwardly extending wall 758 of the spool 734 may extend across the interior lumen 738 thereof to substantially fluidly isolate the first end 746 of the spool 734 from the second end of the spool 750. A small aperture 760 may extend through a thickness of the radially inwardly extending wall 758 to allow air and/or fluid to pass through the aperture 760 to prime the spool valve 700. However, the aperture 760 may be sized to preclude a flow of liquid fluid therethrough. Alternatively, or additionally, the aperture 760 may include a hydrophobic membrane disposed across the opening thereof. The hydrophobic membrane may allow air to pass through the aperture 760 while precluding a flow of water or saline therethrough. Alternatively, the aperture 760 may be sized or configured as a leak channel to allow a small amount of fluid received at the fluid inlet port 706 to pass into the interior lumen 738 of the spool 734 and to the lumen 728 of the effluent outlet port 712. This may allow pressure to dissipate when the roller pump 240 is turned off and the valve to move from the open to the closed configuration, as will be described in more detail herein.

A spring 762 or other biasing mechanism may be disposed within the interior lumen 726 of the valve body 710, such as opposite the fluid inlet port 706. The spring 762 may extend from a first end 764 configured to be positioned adjacent to and/or in contact with a second end portion 768 of the spool 734 to a second end 766 configured to be positioned adjacent to and/or in contact with an interior surface 770 of the valve cap 714 of the spool valve 700. In the absence of an external force, the spring 762 may be configured to bias the spool 734 towards the first or closed configuration. The valve cap 714 may be configured to threadably engage the valve body 710 via a set of mating threads 772, 774. The valve cap 714 may be provided as a separate component which is assembled with the valve body 710. While the valve cap 714 is shown and described has having a threaded coupling 772, 774 with the valve body 710, the valve cap 714 may be secured to the valve body 710 using any means desired such as, but not limited to, adhesives, friction fits, mechanical engagements, welding, soldering, brazing, threading, etc. The valve cap 714 may have an outer diameter that is similar to the outer diameter of the first end region of the valve body 710. The valve cap 714 may include internal threading 772 configured to engage outer threading 774 formed on an outer surface of the valve body 710. In other embodiments, at least one of the fluid inlet port 706 and/or effluent inlet port 708 may be formed as separate components or coupled to separate components that are subsequently assembled with the valve body 710 in a manner similar to the valve cap 714.

The spool 734 may be actuated to the open configuration, shown in FIG. 19, through a flow of fluid passing through the fluid inlet port 706. For example, the fluid inlet port 706 may define a lumen 776 extending from a first end 778 external to the valve body to a second end 780 disposed interior to the valve body 710. The fluid inlet port 706 may extend along or co-linear to the longitudinal axis 724 of the spool valve 700. The lumen 776 may be in fluid communication with the interior lumen 726 of the valve body 710. Fluid may flow through the fluid inlet tube 490 and into the lumen 776 of the fluid inlet port 706 into the interior lumen 726. Once sufficient fluid pressure has accumulated within the interior lumen 726 against a first side of the radially inwardly extending wall 758 of the spool 734, the fluid may push the spool 734 towards the second end 704 of the spool valve 700 and compress the spring 762 to move the spool 734 from the first, closed configuration (FIG. 18) to the second, open configuration (FIG. 19). For example, sufficient fluid pressure may be the amount of pressure required to overcome the biasing force of the spring 762. As the spool 734 is actuated towards the second end 704, the second end 750 of the spool 734 may contact an annular wall 782 of the valve cap 714 to provide a mechanical stop which is spaced to align the intermediate region 754 with the lumen 718 of the effluent inlet port 708. In some instances, the lumen 728 of the effluent outlet port 712 may allow air to escape from the interior lumen 726 as the spool 734 is moved towards the second end 704 of the spool valve 700. Further, the lumen 728 of the effluent outlet port 712 may allow air to enter the interior lumen 726 as the spool 734 is moved towards the first end 702 of the spool valve 700.

The spool valve 700 may be configured to open (e.g., actuate the spool 734) substantially simultaneously with the use of the thrombectomy catheter 58. For example, when the clinician activates the drive unit 12 (e.g., operates a foot switch (not explicitly shown) on the drive unit 12), the pump 56 and the roller pump 240 are both activated. In some instances, the pump 56 may be automatically activated simultaneously or sequentially with the roller pump 240 when the clinician activates the drive unit 12. As a portion 494 of the fluid inlet tube 490 is disposed within the roller pump 240, actuation of the roller pump 240 drives fluid through the fluid inlet tube 490 and into the interior lumen 726 of the valve body 710 via the fluid inlet port 706. As described above, the fluid may be saline, ambient air, or other fluid. The fluid pushes the spool 734 to the open configuration (FIG. 19) and allows for the free flow of effluent through the effluent waste tube 68, through the spool valve 700 along flow path 756 from the effluent inlet port 708 to the effluent outlet port 712, and into the effluent collection bag 28. For example, the flow of effluent is not limited by the speed of the roller pump 240. Rather, effluent may flow freely at a rate that achieves fluid flow balance within the system. As there is no fluid outlet adjacent to the first end 746 of the spool 734, the fluid flow may deadhead the roller pump 240 and no additional fluid is transferred into the interior lumen 726 of the spool valve 700, maintaining the spool 734 in the open position. When the clinician deactivates the drive unit 12 (e.g., releases the foot switch) to stop operation of the thrombectomy catheter 58), the pump 56 and the roller pump 240 are deactivated, stopping fluid inflow into the thrombectomy catheter 58. As the roller pump 240 is not a perfect pump, pressure may dissipate from the interior lumen 726 and the spring 762 may once again bias the spool 734 towards the first end 702 and the closed configuration to stop a flow of effluent through the valve 700 to the effluent waste bag 28.

A small tubular member, such as a hypotube 790 may be positioned in the lumen of the fluid inlet tube 490 at least throughout the portion of the fluid inlet tube 490 passing through the roller pump 240. In some examples, the hypotube 790 may extend into the lumen 776 of the fluid inlet port 706. The placement of the hypotube 790 within the lumen of the tube 490 may provide a lumen or fluid path 792 (e.g., the lumen of the hypotube 790) passing through the hypotube 790 from a location upstream of the roller pump 240 to a location downstream of the roller pump 240. The hypotube 790 may be constructed (e.g., possess sufficient hoop strength) such that the lumen of the hypotube is not collapsed or occluded within the roller pump 240. Accordingly, a small leak channel may be formed from the lumen 726 of the valve 700 through the lumen 792 of the hypotube 790 and past the roller pump 240 such that fluid in the lumen 726 may pass in a retrograde direction through the lumen 792 of the hypotube 790 when the roller pump 240 is deactivated or stopped. In some examples, the hypotube 790 may prevent the rollers 242 from completely occluding the collapsed or compressed lumen of the tube 490 between the roller 242 and the housing 246, leaving an additional leak channel similar in form and function to the leak channel 510 described with respect to FIG. 12.

It is further contemplated that the spool valve 700 may include any of the pressure dissipation features or structures described herein. For instance, it is further contemplated that the aperture 760 may be configured to define a leak channel through the radially inwardly extending wall 758 to allow a small amount of fluid to pass from the first side of the radially inwardly extending wall 758 and into the interior lumen 738 of the spool 734 after the roller pump 240 is deactivated to dissipate pressure from the interior lumen 726. The aperture 760 may be sized such that so that a pressure head may be maintained against the spool 734 to actuate the spool 734 to the open position when the roller pump 240 is running, yet bleed off fluid in the lumen 738 to release the pressure and thus close the valve 700 (i.e., allow the spool 734 to move to the closed position) when the roller pump 240 is stopped. For example, the aperture 760 may have a diameter in the range of about 0.1 millimeters to about 0.2 millimeters.

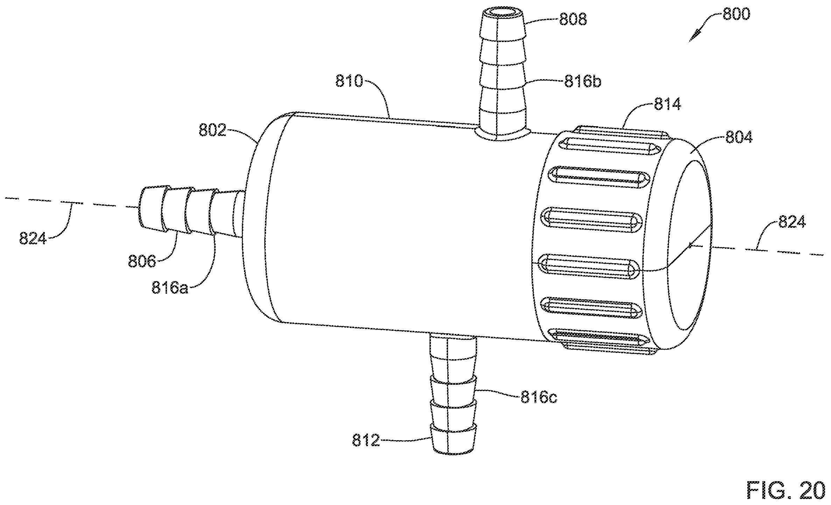

FIG. 20 is a perspective view of another illustrative spool valve 800. The spool valve 800 may include a valve body 810, which in some instances may have a generally tubular cylindrical structure, extending from a first end 802 to a second end 804. A fluid inlet port 806 may extend from the first end 802. The fluid inlet port 806 may be configured to be fluidly coupled to a fluid inlet tube, such as the fluid inlet tube 490 described with respect to FIG. 5. For example, the fluid inlet port 806 may be configured such that the fluid inlet tube 490 may be disposed over and surround the fluid inlet port 806 or may be configured such that the fluid inlet tube 490 extends within the fluid inlet port 806, as desired. In other instances, the fluid inlet tube 490 may be threadably connected to the fluid inlet port 806. The fluid inlet port 806 may be formed as a single monolithic structure with the valve body 810. In other embodiments, the fluid inlet port 806 may be removably coupled to the valve body 810 using, for example, a removable threaded fitting, press-fit, snap fit, friction fit, snap rings, etc., may be used, as desired. In some embodiments, the fluid inlet port 806 may include a tube barb including one or more raised ridges 816a. The raised ridges 816a may increase in diameter towards the valve body 810. This may facilitate assembly of the fluid inlet tube 490 with the fluid inlet port 806 while hindering accidental removal of the fluid inlet tube 490 from the fluid inlet port 806.

An effluent inlet port 808 and an effluent outlet port 812 may extend from the valve body 810, such as from the curved side wall of the valve body 810. Each of the effluent inlet port 808 and the effluent outlet port 812 may be configured to be fluidly coupled to portions of the effluent waste tube 68. A first portion of the effluent waste tube 68 may be fluidly coupled to the effluent inlet port 808 (e.g., disposed over the effluent inlet port 808 or within the effluent inlet port 808) and extend therefrom, as desired. A second portion of the effluent waste tube 68 may be fluidly coupled to the effluent outlet port 812 (e.g., disposed over the effluent outlet port 812 or within the effluent outlet port 812) and extend therefrom, as desired. The effluent inlet port 808 and the effluent outlet port 812 may be fluidly isolated from the fluid inlet port 806. In some cases, the effluent inlet port 808 and the effluent outlet port 812 may be substantially fluidly isolated from the fluid inlet port 806. For example, when the effluent inlet port 808 and the effluent outlet port 812 are substantially fluidly isolated from the fluid inlet port 806, a small pressure dissipation fluid path may extend through the spool valve 800 from the fluid inlet port 806 to the effluent inlet port 808 and/or the effluent outlet port 812 that allows pressure in the fluid inlet tube 490 to dissipate from the spool valve 800 to close the spool valve 800, although this is not required.

In some instances, the effluent inlet port 808 and the effluent outlet port 812 may be positioned on opposite sides of the valve body 810, such as approximately 180° from one another. In some instances, the effluent inlet port 808 may extend parallel to, but axially displaced from the effluent outlet port 812. In some instances, the effluent inlet port 808 and the effluent outlet port 812 may be axially displaced from one another along a longitudinal axis 824 of the valve body 810 such that they do not share a common axis. For example, the effluent inlet port 808 may be positioned closer to the second end 804 of the valve body 810 than the effluent outlet port 812. Alternatively, the effluent inlet port 808 may be positioned closer to the first end 802 of the valve body 810 than the effluent outlet port 812. Other positions and/or configurations of the effluent inlet port 808 and the effluent outlet port 812 may be used as desired. The effluent inlet port 808 may be formed as a single monolithic structure with the valve body 810. In other embodiments, the effluent inlet port 808 may be removably coupled to the valve body 810 using, for example, a removable threaded fitting, press-fit, snap fit, friction fit, snap rings, etc., may be used, as desired. Similarly, the effluent outlet port 812 may be formed as a single monolithic structure with the valve body 810. In other embodiments, the effluent outlet port 812 may be removably coupled to the valve body 810 using, for example, a removable threaded fitting, press-fit, snap fit, friction fit, snap rings, etc., may be used, as desired. One or both of the effluent inlet port 808 and the effluent outlet port 812 may include one or more raised ridges 816b, 816c. The raised ridges 816b, 816c may increase in diameter towards the valve body 810. This may facilitate assembly of the effluent waste tube 68 with the effluent inlet port 808 and/or the effluent outlet port 812 while hindering accidental removal of the effluent waste tube 68 from the effluent inlet port 808 and/or the effluent outlet port 812.

As described above, the spool valve 800 may be positioned in line with the effluent waste tube 68. For instance, the effluent inlet port 808 may be positioned downstream of the connection manifold assembly 62 and the effluent outlet port 812 may be positioned upstream of the effluent collection bag 28. For example, effluent may flow from the thrombectomy catheter 58 through the effluent return tube 66, through the connection manifold assembly 62, and into the effluent waste tube 68. Once in the effluent waste tube 68, the effluent may pass through the spool valve 800 before then entering the effluent collection bag 28.

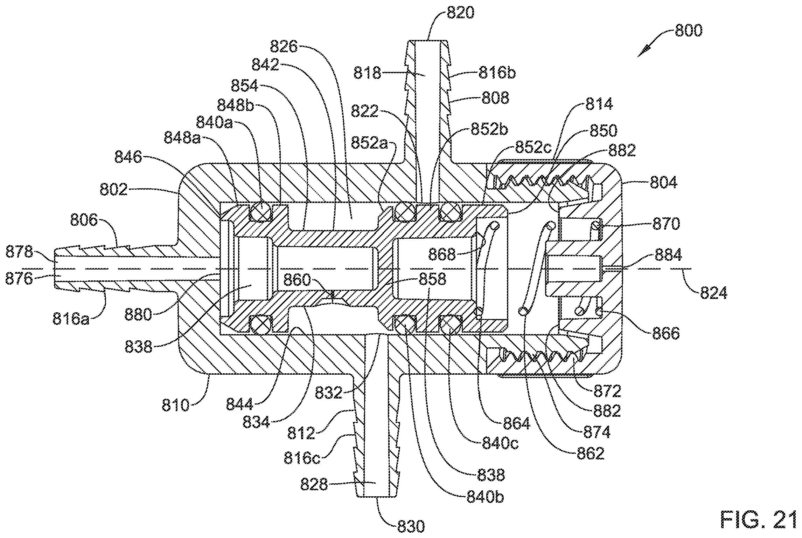

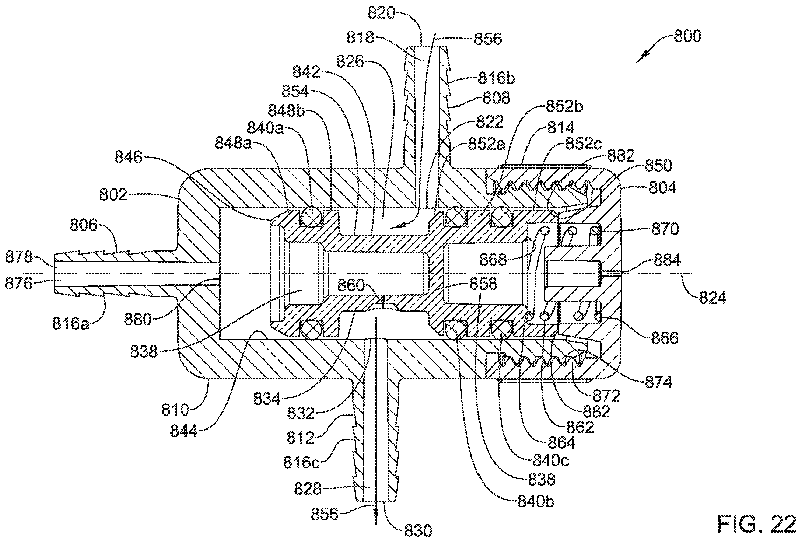

Referring additionally to FIG. 21, which is a cross-sectional view of the spool valve 800 in a first or closed configuration, and referring to FIG. 22, which is a cross-sectional view of the spool valve 800 in a second or open configuration, the effluent inlet port 808 may define a lumen 818 extending from a first end 820 external to the valve body 810 to a second end 822 disposed interior to the valve body 810. The lumen 818 of the effluent inlet port 808 may extend through a side wall of the valve body 810 in a direction generally orthogonal to the longitudinal axis 824 of the spool valve 800 to define an opening from an exterior of the valve body 810 to an interior chamber or lumen 826 of the valve body 810. The lumen 818 of the effluent inlet port 808 may be in selective fluid communication with the lumen 826 of the valve body 810. Similarly, the effluent outlet port 812 may define a lumen 828 extending from a first end 830 external to the valve body 810 to a second end 832 disposed interior to the valve body 810. The lumen 828 of the effluent outlet port 812 may extend through a side wall of the valve body 810 in a direction generally orthogonal to the longitudinal axis 824 of the spool valve 800 to define an opening from an exterior of the valve body 810 to an interior chamber or lumen 826 of the valve body 810.

A generally cylindrical spool or piston 834 may be slidably disposed within the interior lumen 826 of the valve body 810. The spool 834 may be actuatable along or co-linear with the longitudinal axis 824 of the spool valve 800. In a first or closed configuration (FIG. 21), the spool 834 may be positioned to block or close off the lumen 818 of the effluent inlet port 808. This may prevent effluent from entering the lumen 826 of the valve body 810. In a second or open configuration (FIG. 22), the spool 834 may be displaced along the longitudinal axis 824 to align a fluid pathway through the spool 834 with the lumen 818 to fluidly connect the lumen 818 of the effluent inlet port 808 with the lumen 828 of the effluent outlet port 812.

The spool 834 may vary in diameter over a length thereof. For example, the spool 834 may include an intermediate region 854, or annular groove, having a first outer diameter, longitudinally spaced raised regions 848a, 848b (collectively, 848) adjacent a first end 846 of the spool 834, and longitudinally spaced raised regions 852a, 852b (collectively, 852) adjacent a second end 850 of the spool 834. Each of the intermediate region 854 and the raised regions 848, 852 may extend about a circumference of the spool 834. The raised regions 848, 852 may have an outer diameter that is greater that the first outer diameter of the intermediate region 854. In some examples, the raised regions 848, 852 may have an outer diameter that is substantially the same as the inner diameter of the interior lumen 826 so as to form a fluid tight seal at the lumen 818 of the effluent inlet port 808 when the spool 834 is in the closed configuration. However, in other embodiments, the raised regions 848, 852 may have an outer diameter that is less than the inner diameter of the interior lumen 826. In some embodiments, the spool 834 may be formed from a resilient material, such as, but not limited to, rubber, silicone, etc. to allow portions of the spool 834 to contact an inner surface of the valve body 810 and create a fluid tight seal.

The spool 834 may include a plurality of O-rings or other sealing members 840a, 840b, 840c (collectively, 840) positioned at intervals along a length of the spool 834. The O-rings 840 may be positioned between an outer surface 842 of the spool 834 and an inner surface 844 of the valve body 810 to provide a fluid tight seal between the spool 834 and the valve body 810. A first O-ring 840a may be positioned adjacent to the first end 846 of the spool 834. The first O-ring 840a may be positioned within a recess defined by a pair of raised regions 848a, 848b. The first O-ring 840a may be configured to substantially fluidly isolate the fluid inlet port 806 from the effluent inlet port 808 and the effluent outlet port 812. A second O-ring 840b and a third O-ring 840c may be positioned adjacent to the second end 850 of the spool 834. The second and third O-rings 840b, 840c may be positioned within recesses defined by a plurality of raised regions 852a, 852b, 852c. When the spool valve 800 is in the closed configuration (FIG. 21), the second and third O-rings 840b, 840c may be positioned on a first side and a second side, respectively, of the second end 822 of the lumen 818 of the effluent inlet port 808 such that the lumen 818 of the effluent inlet port 808 is positioned between the second and third O-rings 840b, 840c. The second and third O-rings 840b, 840c may fluidly isolate the lumen 818 of the effluent inlet port 808 from the lumen 826 of the valve body 810 when the valve 800 is in the closed configuration. For example, when the valve 800 is in the closed configuration, fluid may not exit the lumen 818 of the effluent inlet port 808.

The intermediate region 854 of the spool 834 may have an outer diameter less than the diameter of the raised regions 848, 852 that may be moved into alignment with the lumen 818 of the effluent inlet port 808 in the second, or open configuration (FIG. 22). It is noted that in the first, or closed configuration, the intermediate region 854 of the spool 834 may be moved out of alignment with the lumen 818 of the effluent inlet port 808 to fluidly isolate the effluent inlet port 808 from the effluent outlet port 812 via the second and third O-rings 840b, 840c. Movement of the spool 834 to align the intermediate region 854 with the lumen 818 of the effluent inlet port 808 may fluidly couple the lumen 818 of the effluent inlet port 808 with the lumen 828 of the effluent outlet port 812. For example, as fluid passes through the lumen 818 of the effluent inlet port 808, the fluid may enter the lumen 826 of the valve body 810 and the annular space between the outer surface 842 of the spool 834 and the inner surface 844 of the valve body 810. The lumen 828 of the effluent outlet port 812 may also be in fluid communication with the annular space between the outer surface 842 of the spool 834 and the inner surface 844 of the valve body 810. Thus, fluid may flow out of the lumen 826 of the valve body 810 via the lumen 828 of the effluent outlet port 812, as shown at flow path 856.

It is contemplated that a radially inwardly extending wall 858 of the spool 834 may extend across the interior lumen 838 thereof to substantially fluidly isolate the first end 846 of the spool 834 from the second end of the spool 850. A small aperture 860 may extend through a thickness of a wall of the spool 850, such as through the circumferential wall of the intermediate region 854 of the spool 834 to allow air and/or fluid to pass through the aperture 860 to prime the spool valve 800. However, the aperture 860 may be sized to preclude a flow of liquid fluid therethrough. Alternatively, or additionally, the aperture 860 may include a hydrophobic membrane disposed across the opening thereof. The hydrophobic membrane may allow air to pass through the aperture 860 while precluding a flow of water or saline therethrough. Alternatively, the aperture 860 may be sized or configured as a leak channel to allow a small amount of fluid received at the fluid inlet port 806 to pass through the circumferential wall of the intermediate region 854 of the spool 834 and to the lumen 828 of the effluent outlet port 812. This may allow pressure to dissipate when the roller pump 240 is turned off and the valve to move from the open to the closed configuration, as will be described in more detail herein.

A spring 862 or other biasing mechanism may be disposed within the interior lumen 826 of the valve body 810, such as opposite the fluid inlet port 806. The spring 862 may extend from a first end 864 configured to be positioned adjacent to and/or in contact with a second end portion 868 of the spool 834 to a second end 866 configured to be positioned adjacent to and/or in contact with an interior surface 870 of a valve cap 814 of the spool valve 800. In the absence of an external force, the spring 862 may be configured to bias the spool 834 towards the first or closed configuration. The valve cap 814 may be releasably coupled to the valve body 810. For example, the valve cap 814 may be configured to threadably engage the valve body 810 via a set of mating threads 872, 874. The valve cap 814 may be provided as a separate component which is assembled with the valve body 810. While the valve cap 814 is shown and described has having a threaded coupling 872, 874 with the valve body 810, the valve cap 814 may be secured to the valve body 810 using any means desired such as, but not limited to, adhesives, friction fits, mechanical engagements, welding, soldering, brazing, threading, etc. The valve cap 814 may have an outer diameter that is similar to the outer diameter of the first end region of the valve body 810. The valve cap 814 may include internal threading 872 configured to engage outer threading 874 formed on an outer surface of the valve body 810. In other embodiments, at least one of the fluid inlet port 806 and/or effluent inlet port 808 may be formed as separate components or coupled to separate components that are subsequently assembled with the valve body 810 in a manner similar to the valve cap 814.

The spool 834 may be actuated to the open configuration, shown in FIG. 22, through a flow of fluid passing through the fluid inlet port 806. For example, the fluid inlet port 806 may define a lumen 876 extending from a first end 878 external to the valve body to a second end 880 disposed interior to the valve body 810. The fluid inlet port 806 may extend along or co-linear to the longitudinal axis 824 of the spool valve 800. The lumen 876 may be in fluid communication with the interior lumen 826 of the valve body 810. Fluid may flow through the fluid inlet tube 490 and into the lumen 876 of the fluid inlet port 806 into the interior lumen 826. Once sufficient fluid pressure has accumulated within the interior lumen 826 against a first side of the radially inwardly extending wall 858 of the spool 834, the fluid may push the spool 834 towards the second end 804 of the spool valve 800 and compress the spring 862 to move the spool 834 from the first, closed configuration (FIG. 21) to the second, open configuration (FIG. 22). For example, sufficient fluid pressure may be the amount of pressure required to overcome the biasing force of the spring 862. As the spool 834 is actuated towards the second end 804, the second end 850 of the spool 834 may contact an annular wall 882 of the valve cap 814 to provide a mechanical stop which is spaced to align the intermediate region 854 with the lumen 818 of the effluent inlet port 808. In some instances, a through hole 884 may extend through the valve cap 814 to allow air to escape from the interior lumen 826 as the spool 834 is moved towards the second end 804 of the spool valve 800. Further, the through hole 884 may allow air to enter the interior lumen 826 as the spool 834 is moved towards the first end 802 of the spool valve 800.

The spool valve 800 may be configured to open (e.g., actuate the spool 834) substantially simultaneously with the use of the thrombectomy catheter 58. For example, when the clinician activates the drive unit 12 (e.g., operates a foot switch (not explicitly shown) on the drive unit 12), the pump 56 and the roller pump 240 are both activated. In some instances, the pump 56 may be automatically activated simultaneously or sequentially with the roller pump 240 when the clinician activates the drive unit 12. As a portion 494 of the fluid inlet tube 490 is disposed within the roller pump 240, actuation of the roller pump 240 drives fluid through the fluid inlet tube 490 and into the interior lumen 826 of the valve body 810 via the fluid inlet port 806. As described above, the fluid may be saline, ambient air, or other fluid. The fluid pushes the spool 834 to the open configuration (FIG. 22) and allows for the free flow of effluent through the effluent waste tube 68, through the spool valve 800 along flow path 856 from the effluent inlet port 808 to the effluent outlet port 812, and into the effluent collection bag 28. For example, the flow of effluent is not limited by the speed of the roller pump 240. Rather, effluent may flow freely at a rate that achieves fluid flow balance within the system. As there is no fluid outlet adjacent to the first end 846 of the spool 834, the fluid flow may deadhead the roller pump 240 and no additional fluid is transferred into the interior lumen 826 of the spool valve 800, maintaining the spool 834 in the open position. When the clinician deactivates the drive unit 12 (e.g., releases the foot switch) to stop operation of the thrombectomy catheter 58), the pump 56 and the roller pump 240 are deactivated, stopping fluid inflow into the thrombectomy catheter 58. As the roller pump 240 is not a perfect pump, pressure may dissipate from the interior lumen 826 and the spring 862 may once again bias the spool 834 towards the first end 802 and the closed configuration to stop a flow of effluent through the valve 800 to the effluent waste bag 28.

It is further contemplated that the spool valve 800 may include any of the pressure dissipation features or structures described herein. For instance, it is further contemplated that the aperture 860 may be configured to define a leak channel through the circumferential wall of the intermediate region 854 to allow a small amount of fluid to pass therethrough from the lumen 876of the fluid inlet port 806 to the lumen 828 of the effluent outlet port 812 after the roller pump 240 is deactivated to dissipate pressure from the interior lumen 726. The aperture 860 may be sized such that so that a pressure head may be maintained against the spool 834 to actuate the spool 834 to the open position when the roller pump 240 is running, yet bleed off fluid in the lumen 738 to release the pressure and thus close the valve 800 (i.e., allow the spool 834 to move to the closed position) when the roller pump 240 is stopped. For example, the aperture 860 may have a diameter in the range of about 0.1 millimeters to about 0.2 millimeters.

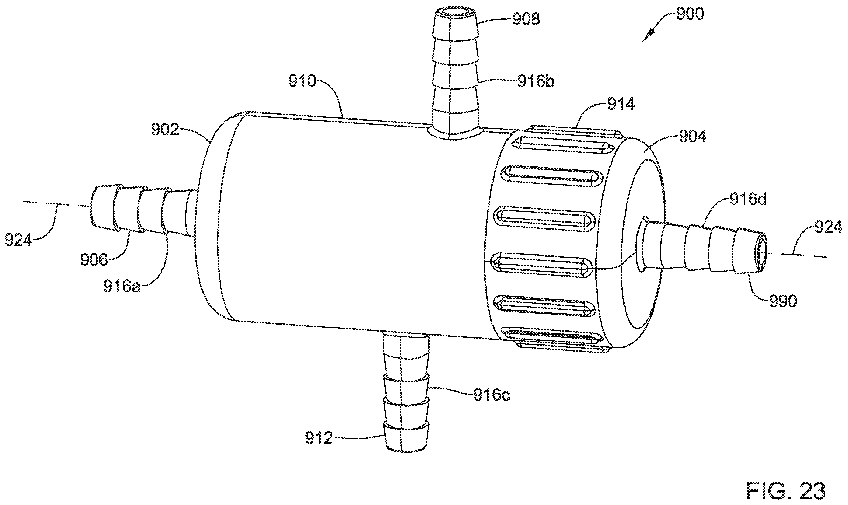

FIG. 23 is a perspective view of another illustrative spool valve 900. The spool valve 900 may include a valve body 910, which in some instances may have a generally tubular cylindrical structure, extending from a first end 902 to a second end 904. A fluid inlet port 906 may extend from the first end 902. The fluid inlet port 906 may be configured to be fluidly coupled to a fluid inlet tube, such as the fluid inlet tube 490 described with respect to FIG. 5. For example, the fluid inlet port 906 may be configured such that the fluid inlet tube 490 may be disposed over and surround the fluid inlet port 906 or may be configured such that the fluid inlet tube 490 extends within the fluid inlet port 906, as desired. In other instances, the fluid inlet tube 490 may be threadably connected to the fluid inlet port 906. The fluid inlet port 906 may be formed as a single monolithic structure with the valve body 910. In other embodiments, the fluid inlet port 906 may be removably coupled to the valve body 910 using, for example, a removable threaded fitting, press-fit, snap fit, friction fit, snap rings, etc., may be used, as desired. In some embodiments, the fluid inlet port 906 may include a tube barb including one or more raised ridges 916a. The raised ridges 916a may increase in diameter towards the valve body 910. This may facilitate assembly of the fluid inlet tube 490 with the fluid inlet port 906 while hindering accidental removal of the fluid inlet tube 490 from the fluid inlet port 906.

A fluid outlet port 990 may extend from the second end 904. The fluid outlet port 990 may be configured to be fluidly coupled to a fluid outlet tube (not explicitly shown) (e.g., disposed over the effluent inlet port 908 or within the effluent inlet port 908) and extend therefrom, as desired. The fluid outlet tube may in turn be fluidly coupled to a second waste collection bag (not explicitly shown). The second waste collection bag may be separate from and provided in addition to the waste collection bag 28 described herein. In some instances, the fluid outlet tube may be threadably connected to the fluid outlet port 990. In some embodiments, the fluid outlet port 990 may be removably coupled to the valve body 910 using, for example, a removable threaded valve cap 914. Other coupling means, such as, but not limited to, press-fit, snap fit, friction fit, snap rings, etc., may be used, as desired. In yet other examples, the fluid outlet port 990 may be formed as a single monolithic structure with the valve body 910. In some embodiments, the fluid outlet port 990 may include a tube barb including one or more raised ridges 916d. The raised ridges 916d may increase in diameter towards the valve body 910. This may facilitate assembly of the fluid outlet tube 490 with the fluid outlet port 990 while hindering accidental removal of the fluid outlet tube from the fluid outlet port 990. The fluid outlet port 990 may be in fluid communication with the fluid inlet port 906 via an aperture 960, as will be described in more detail herein.

An effluent inlet port 908 and an effluent outlet port 912 may extend from the valve body 910, such as from the curved side wall of the valve body 910. Each of the effluent inlet port 908 and the effluent outlet port 912 may be configured to be fluidly coupled to portions of the effluent waste tube 68. A first portion of the effluent waste tube 68 may be fluidly coupled to the effluent inlet port 908 (e.g., disposed over the effluent inlet port 908 or within the effluent inlet port 908) and extend therefrom, as desired. A second portion of the effluent waste tube 68 may be fluidly coupled to the effluent outlet port 912 (e.g., disposed over the effluent outlet port 912 or within the effluent outlet port 912) and extend therefrom, as desired. The effluent inlet port 908 and the effluent outlet port 912 may be fluidly isolated from the fluid inlet port 906 and the fluid outlet port 990. In some cases, the effluent inlet port 908 and the effluent outlet port 912 may be substantially fluidly isolated from the fluid inlet port 906 and the fluid outlet port 990. For example, when the effluent inlet port 908 and the effluent outlet port 912 are substantially fluidly isolated from the fluid inlet port 906 and the fluid outlet port 990, a small pressure dissipation fluid path may extend through the spool valve 900 from the fluid inlet port 906 to the effluent inlet port 908 and/or the effluent outlet port 912 that allows pressure in the fluid inlet tube 490 to dissipate from the spool valve 900 to close the spool valve 900, although this is not required.

In some instances, the effluent inlet port 908 and the effluent outlet port 912 may be positioned on opposite sides of the valve body 910, such as approximately 180° from one another. In some instances, the effluent inlet port 908 may extend parallel to, but axially displaced from the effluent outlet port 912. In some instances, the effluent inlet port 908 and the effluent outlet port 912 may be axially displaced from one another along a longitudinal axis 924 of the valve body 910 such that they do not share a common axis. For example, the effluent inlet port 908 may be positioned closer to the second end 904 of the valve body 910 than the effluent outlet port 912. Alternatively, the effluent inlet port 908 may be positioned closer to the first end 902 of the valve body 910 than the effluent outlet port 912. Other positions and/or configurations of the effluent inlet port 908 and the effluent outlet port 912 may be used as desired. The effluent inlet port 908 may be formed as a single monolithic structure with the valve body 910. In other embodiments, the effluent inlet port 908 may be removably coupled to the valve body 910 using, for example, a removable threaded fitting, press-fit, snap fit, friction fit, snap rings, etc., may be used, as desired. Similarly, the effluent outlet port 912 may be formed as a single monolithic structure with the valve body 910. In other embodiments, the effluent outlet port 912 may be removably coupled to the valve body 910 using, for example, a removable threaded fitting, press-fit, snap fit, friction fit, snap rings, etc., may be used, as desired. One or both of the effluent inlet port 908 and the effluent outlet port 912 may include one or more raised ridges 916b, 916c. The raised ridges 916b, 916c may increase in diameter towards the valve body 910. This may facilitate assembly of the effluent waste tube 68 with the effluent inlet port 908 and/or the effluent outlet port 912 while hindering accidental removal of the effluent waste tube 68 from the effluent inlet port 908 and/or the effluent outlet port 912.

As described above, the spool valve 900 may be positioned in line with the effluent waste tube 68. For instance, the effluent inlet port 908 may be positioned downstream of the connection manifold assembly 62 and the effluent outlet port 912 may be positioned upstream of the effluent collection bag 28. For example, effluent may flow from the thrombectomy catheter 58 through the effluent return tube 66, through the connection manifold assembly 62, and into the effluent waste tube 68. Once in the effluent waste tube 68, the effluent may pass through the spool valve 900 before then entering the effluent collection bag 28.

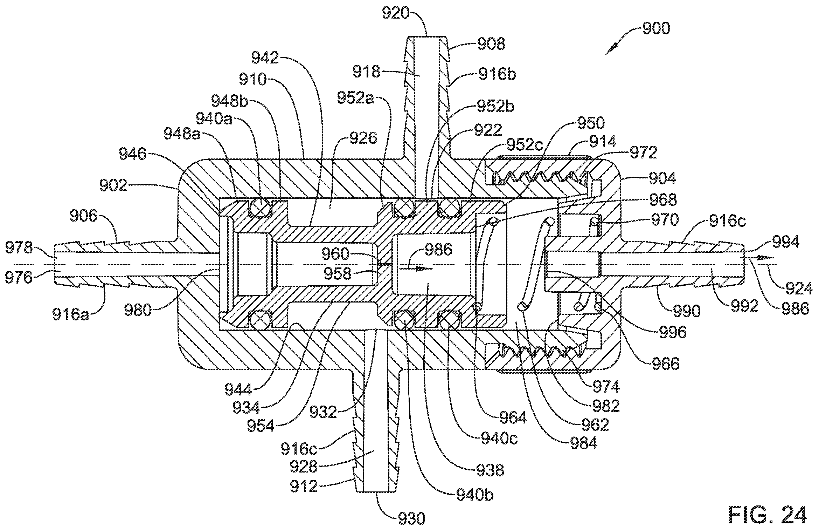

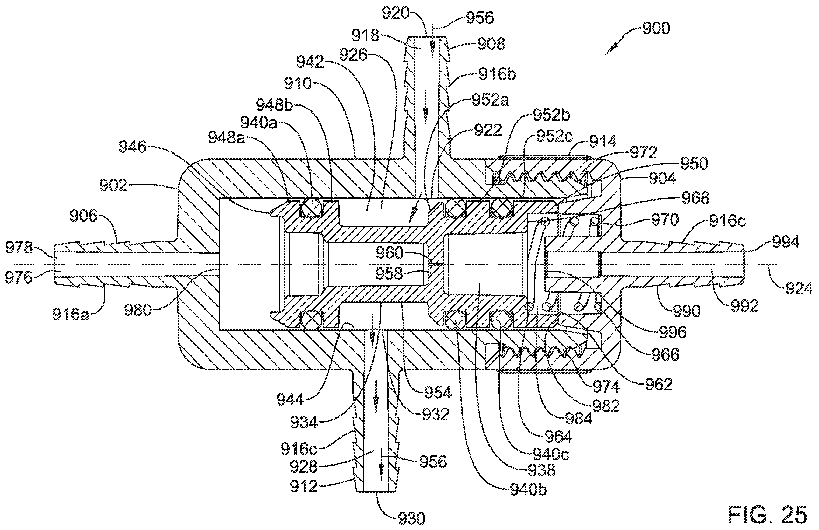

Referring additionally to FIG. 24, which is a cross-sectional view of the spool valve 900 in a first or closed configuration, and referring to FIG. 25, which is a cross-sectional view of the spool valve 900 in a second or open configuration, the effluent inlet port 908 may define a lumen 918 extending from a first end 920 external to the valve body 910 to a second end 922 disposed interior to the valve body 910. The lumen 918 of the effluent inlet port 908 may extend through a side wall of the valve body 910 in a direction generally orthogonal to the longitudinal axis 924 of the spool valve 900 to define an opening from an exterior of the valve body 910 to an interior chamber or lumen 926 of the valve body 910. The lumen 918 of the effluent inlet port 908 may be in selective fluid communication with the lumen 926 of the valve body 910. Similarly, the effluent outlet port 912 may define a lumen 928 extending from a first end 930 external to the valve body 910 to a second end 932 disposed interior to the valve body 910. The lumen 928 of the effluent outlet port 912 may extend through a side wall of the valve body 910 in a direction generally orthogonal to the longitudinal axis 924 of the spool valve 900 to define an opening from an exterior of the valve body 910 to an interior chamber or lumen 926 of the valve body 910.

A generally cylindrical spool or piston 934 may be slidably disposed within the interior lumen 926 of the valve body 910. The spool 934 may be actuatable along or co-linear with the longitudinal axis 924 of the spool valve 900. In a first or closed configuration (FIG. 24), the spool 934 may be positioned to block or close off the lumen 918 of the effluent inlet port 908. This may prevent effluent from entering the lumen 926 of the valve body 910. In a second or open configuration (FIG. 25), the spool 934 may be displaced along the longitudinal axis 924 to align a fluid pathway through the spool 934 with the lumen 918 to fluidly connect the lumen 918 of the effluent inlet port 908 with the lumen 928 of the effluent outlet port 912.

The spool 934 may vary in diameter over a length thereof. For example, the spool 934 may include an intermediate region 954, or annular groove, having a first outer diameter, longitudinally spaced raised regions 948a, 948b (collectively, 948) adjacent a first end 946 of the spool 934, and longitudinally spaced raised regions 952a, 952b (collectively, 952) adjacent a second end 950 of the spool 934. Each of the intermediate region 954 and the raised regions 948, 952 may extend about a circumference of the spool 934. The raised regions 948, 952 may have an outer diameter that is greater that the first outer diameter of the intermediate region 954. In some examples, the raised regions 948, 952 may have an outer diameter that is substantially the same as the inner diameter of the interior lumen 926 so as to form a fluid tight seal at the lumen 918 of the effluent inlet port 908 when the spool 934 is in the closed configuration. However, in other embodiments, the raised regions 948, 952 may have an outer diameter that is less than the inner diameter of the interior lumen 926. In some embodiments, the spool 934 may be formed from a resilient material, such as, but not limited to, rubber, silicone, etc. to allow portions of the spool 934 to contact an inner surface of the valve body 910 and create a fluid tight seal.

The spool 934 may include a plurality of O-rings or other sealing members 940a, 940b, 940c (collectively, 940) positioned at intervals along a length of the spool 934. The O-rings 940 may be positioned between an outer surface 942 of the spool 934 and an inner surface 944 of the valve body 910 to provide a fluid tight seal between the spool 934 and the valve body 910. A first O-ring 940a may be positioned adjacent to the first end 946 of the spool 934. The first O-ring 940a may be positioned within a recess defined by a pair of raised regions 948a, 948b. The first O-ring 940a may be configured to substantially fluidly isolate the fluid inlet port 906 from the effluent inlet port 908 and the effluent outlet port 912. A second O-ring 940b and a third O-ring 940c may be positioned adjacent to the second end 950 of the spool 934. The second and third O-rings 940b, 940c may be positioned within recesses defined by a plurality of raised regions 952a, 952b, 952c. When the spool valve 900 is in the closed configuration (FIG. 24), the second and third O-rings 940b, 940c may be positioned on a first side and a second side, respectively, of the second end 922 of the lumen 918 of the effluent inlet port 908 such that the lumen 918 of the effluent inlet port 908 is positioned between the second and third O-rings 940b, 940c. The second and third O-rings 940b, 940c may fluidly isolate the lumen 918 of the effluent inlet port 908 from the lumen 926 of the valve body 910 when the valve 900 is in the closed configuration. For example, when the valve 900 is in the closed configuration, fluid may not exit the lumen 918 of the effluent inlet port 908.

The intermediate region 954 of the spool 934 may have an outer diameter less than the diameter of the raised regions 948, 952 that may be moved into alignment with the lumen 918 of the effluent inlet port 908 in the second, or open configuration (FIG. 25). It is noted that in the first, or closed configuration, the intermediate region 954 of the spool 934 may be moved out of alignment with the lumen 918 of the effluent inlet port 908 to fluidly isolate the effluent inlet port 908 from the effluent outlet port 912 via the second and third O-rings 940b, 940c. Movement of the spool 934 to align the intermediate region 954 with the lumen 918 of the effluent inlet port 908 may fluidly couple the lumen 918 of the effluent inlet port 908 with the lumen 928 of the effluent outlet port 912. For example, as fluid passes through the lumen 918 of the effluent inlet port 908, the fluid may enter the lumen 926 of the valve body 910 and the annular space between the outer surface 942 of the spool 934 and the inner surface 944 of the valve body 910. The lumen 928 of the effluent outlet port 912 may also be in fluid communication with the annular space between the outer surface 942 of the spool 934 and the inner surface 944 of the valve body 910. Thus, fluid may flow out of the lumen 926 of the valve body 910 via the lumen 928 of the effluent outlet port 912, as shown at flow path 956.

It is contemplated that a radially inwardly extending wall 958 of the spool 934 may extend across the interior lumen 938 thereof to substantially fluidly isolate the first end 946 of the spool 934 from the second end of the spool 950. An aperture 960 may extend through a thickness of the radially inwardly extending wall 958 to allow air and/or fluid to pass through the aperture 960 to prime the spool valve 900. However, the aperture 960 may be sized to preclude a flow of liquid fluid therethrough. Alternatively, or additionally, the aperture 960 may include a hydrophobic membrane disposed across the opening thereof. The hydrophobic membrane may allow air to pass through the aperture 960 while precluding a flow of water or saline therethrough.

Alternatively, the aperture 960 may be sized or configured as a leak channel to allow a small amount of fluid received at the fluid inlet port 906 to pass into the interior lumen 938 of the spool 934 and to the lumen 992 of the fluid outlet port 990. This may allow pressure to dissipate when the roller pump 240 is turned off and the valve to move from the open to the closed configuration, as will be described in more detail herein. The radially inwardly extending wall 958 of the spool 934 may be axially positioned to be distal to the intermediate region 958 such that fluid leaking through the aperture 960 does not enter the effluent flow path 956. For example, the second and third O-rings 940b, 940c may fluidly isolate the effluent flow path 956 from a second end region 984 of the lumen 926 of the valve body 910 adjacent the effluent flow path 956.

For example, the fluid inlet port 906 may define a lumen 976 extending from a first end 978 external to the valve body to a second end 980 disposed interior to the valve body 910. The fluid inlet port 906 may extend along or co-linear to the longitudinal axis 924 of the spool valve 900. The lumen 976 may be in fluid communication with the interior lumen 926 of the valve body 910. The fluid outlet port 990 may define a lumen 992 extending from a first end 994 external to the valve body to a second end 996 disposed interior to the valve body 910. The fluid outlet port 990 may extend along or co-linear to the longitudinal axis 924 of the spool valve 900. The lumen 992 may be in fluid communication with the second end region 984 of the interior lumen 926 of the valve body 910. Fluid may flow through the fluid inlet tube 490 and the lumen 976 of the fluid inlet port 906 into the interior lumen 926, seep through the aperture 960, and exit the second end region 984 of the interior lumen 926 via the lumen 992 of the fluid outlet port 990, as shown at arrow 986. The fluid exiting the fluid outlet port 990 may travel through a fluid outlet tube to a second collection bag. It is contemplated that providing a second fluid collection bag to collect fluid from the fluid source may allow for a longer run time of the device. For example, two collection bags may allow the thrombectomy system to operate for a longer time period by providing a larger volume for effluent and/or fluid.

A spring 962 or other biasing mechanism may be disposed within the interior lumen 926 of the valve body 910, such as opposite the fluid inlet port 906. The spring 962 may extend from a first end 964 configured to be positioned adjacent to and/or in contact with a second end portion 968 of the spool 934 to a second end 966 configured to be positioned adjacent to and/or in contact with an interior surface 970 of the valve cap 914 of the spool valve 900. In the absence of an external force, the spring 962 may be configured to bias the spool 934 towards the first or closed configuration. The valve cap 914 may be releasably coupled to the valve body 910. For example, the valve cap 914 may be configured to threadably engage the valve body 910 via a set of mating threads 972, 974. The valve cap 914 may be provided as a separate component which is assembled with the valve body 910. While the valve cap 914 is shown and described has having a threaded coupling 972, 974 with the valve body 910, the valve cap 914 may be secured to the valve body 910 using any means desired such as, but not limited to, adhesives, friction fits, mechanical engagements, welding, soldering, brazing, threading, etc. The valve cap 914 may have an outer diameter that is similar to the outer diameter of the first end region of the valve body 910. The valve cap 914 may include internal threading 972 configured to engage outer threading 974 formed on an outer surface of the valve body 910. In other embodiments, at least one of the fluid inlet port 906, effluent inlet port 908, and/or effluent outlet port 912 may be formed as separate components or coupled to separate components that are subsequently assembled with the valve body 910 in a manner similar to the valve cap 914.

The spool 934 may be actuated to the open configuration, shown in FIG. 25, through a flow of fluid passing through the fluid inlet port 906. Fluid may flow through the fluid inlet tube 490 into the lumen 976 of the fluid inlet port 906 and into the interior lumen 926. Once sufficient fluid pressure has accumulated within the interior lumen 926 against a first side of the radially inwardly extending wall 958 of the spool 934, the fluid may push the spool 934 towards the second end 904 of the spool valve 900 and compress the spring 962 to move the spool 934 from the first, closed configuration (FIG. 24) to the second, open configuration (FIG. 25). For example, sufficient fluid pressure may be the amount of pressure required to overcome the biasing force of the spring 962. As the spool 934 is actuated towards the second end 904, the second end 950 of the spool 934 may contact an annular wall 982 of the valve cap 914 to provide a mechanical stop which is spaced to align the intermediate region 954 with the lumen 918 of the effluent inlet port 908. The lumen 992 of the fluid outlet port 990 may extend through the valve cap 914 and allow air to escape from the interior lumen 926 as the spool 934 is moved towards the second end 904 of the spool valve 900. Further, the lumen 992 of the fluid outlet port 990 may allow air to enter the interior lumen 926 as the spool 934 is moved towards the first end 902 of the spool valve 900.

The spool valve 900 may be configured to open (e.g., actuate the spool 934) substantially simultaneously with the use of the thrombectomy catheter 58. For example, when the clinician activates the drive unit 12 (e.g., operates a foot switch (not explicitly shown) on the drive unit 12), the pump 56 and the roller pump 240 are both activated. In some instances, the pump 56 may be automatically activated simultaneously or sequentially with the roller pump 240 when the clinician activates the drive unit 12. As a portion 494 of the fluid inlet tube 490 is disposed within the roller pump 240, actuation of the roller pump 240 drives fluid through the fluid inlet tube 490 and into the interior lumen 926 of the valve body 910 via the fluid inlet port 906. As described above, the fluid may be saline, ambient air, or other fluid. The fluid pushes the spool 934 to the open configuration (FIG. 25) and allows for the free flow of effluent through the effluent waste tube 68, through the spool valve 900 along flow path 956 from the effluent inlet port 908 to the effluent outlet port 912, and into the effluent collection bag 28. For example, the flow of effluent is not limited by the speed of the roller pump 240. Rather, effluent may flow freely at a rate that achieves fluid flow balance within the system. As there is no fluid outlet adjacent to the first end 946 of the spool 934, the fluid flow may deadhead the roller pump 240 and no additional fluid is transferred into the interior lumen 926 of the spool valve 900, maintaining the spool 934 in the open position. When the clinician deactivates the drive unit 12 (e.g., releases the foot switch) to stop operation of the thrombectomy catheter 58), the pump 56 and the roller pump 240 are deactivated, stopping fluid inflow into the thrombectomy catheter 58. As the roller pump 240 is not a perfect pump, pressure may dissipate from the interior lumen 926 and the spring 962 may once again bias the spool 934 towards the first end 902 and the closed configuration to stop a flow of effluent through the valve 900 to the effluent waste bag 28.

It is further contemplated that the aperture 960 in the radially inwardly extending wall 958 may be configured to define a leak channel to allow a small amount of fluid to pass from the first side of the radially inwardly extending wall 958 and into the second end region 984 of the interior lumen 926 of the valve body 910 after the roller pump 240 is deactivated to dissipate pressure from the interior lumen 926, thus fluidly connecting the fluid inlet port 906 with the fluid outlet port 990. The aperture 960 may be sized such that so that a pressure head may be maintained against the spool 934 to actuate the spool 934 to the open position when the roller pump 240 is running, yet bleed off fluid in the lumen 938 to release the pressure and thus close the valve 900 (i.e., allow the spool 934 to move to the closed position) when the roller pump 240 is stopped. For example, the aperture 960 may have a diameter in the range of about 0.1 millimeters to about 0.2 millimeters. It is further contemplated that the spool valve 900 may include any of the pressure dissipation features or structures described herein.

The materials that can be used for the various components of the thrombectomy catheter, spool valve, pump/catheter assembly, and/or other devices disclosed herein may include those commonly associated with medical devices. For simplicity purposes, the following discussion makes reference to the pump/catheter assembly and its related components. However, this is not intended to limit the devices and methods described herein, as the discussion may be applied to other similar devices, tubular members and/or components of tubular members or devices disclosed herein.

The various components of the devices/systems disclosed herein may include a metal, metal alloy, polymer (some examples of which are disclosed herein), a metalpolymer composite, ceramics, combinations thereof, and the like, or other suitable material. Some examples of suitable metals and metal alloys include stainless steel, such as 304V, 304L, and 316LV stainless steel; mild steel; nickel-titanium alloy such as linear-elastic and/or super-elastic nitinol; other nickel alloys such as nickel-chromium-molybdenum alloys (e.g., UNS: N06625 such as INCONEL^{®} 625, UNS: N06022 such as HASTELLOY^{®} C-22^{®}, UNS: N10276 such as HASTELLOY^{®} C276^{®}, other HASTELLOY^{®} alloys, and the like), nickel-copper alloys (e.g., UNS: N04400 such as MONEL^{®} 400, NICKELVAC^{®} 400, NICORROS^{®} 400, and the like), nickel-cobalt-chromium-molybdenum alloys (e.g., UNS: R30035 such as MP35-N^{®} and the like), nickel-molybdenum alloys (e.g., UNS: N10665 such as HASTELLOY^{®} ALLOY B2^{®}), other nickel-chromium alloys, other nickel-molybdenum alloys, other nickel-cobalt alloys, other nickel-iron alloys, other nickel-copper alloys, other nickel-tungsten or tungsten alloys, and the like; cobalt-chromium alloys; cobalt-chromium-molybdenum alloys (e.g., UNS: R30003 such as ELGILOY^{®}, PHYNOX^{®}, and the like); platinum enriched stainless steel; titanium; combinations thereof; and the like; or any other suitable material.

Some examples of suitable polymers may include polytetrafluoroethylene (PTFE), ethylene tetrafluoroethylene (ETFE), fluorinated ethylene propylene (FEP), polyoxymethylene (POM, for example, DELRIN^{®} available from DuPont), polyether block ester, polyurethane (for example, Polyurethane 85A), polypropylene (PP), polyvinylchloride (PVC), polyether-ester (for example, ARNITEL^{®} available from DSM Engineering Plastics), ether or ester based copolymers (for example, butylene/poly(alkylene ether) phthalate and/or other polyester elastomers such as HYTREL^{®} available from DuPont), polyamide (for example, DURETHAN^{®} available from Bayer or CRISTAMID^{®} available from Elf Atochem), elastomeric polyamides, block polyamide/ethers, polyether block amide (PEBA, for example available under the trade name PEBAX^{®}), ethylene vinyl acetate copolymers (EVA), silicones, polyethylene (PE), MARLEX^{®} high-density polyethylene, MARLEX^{®} lowdensity polyethylene, linear low density polyethylene (for example REXELL^{®}), polyester, polybutylene terephthalate (PBT), polyethylene terephthalate (PET), polytrimethylene terephthalate, polyethylene naphthalate (PEN), polyetheretherketone (PEEK), polyimide (PI), polyetherimide (PEI), polyphenylene sulfide (PPS), polyphenylene oxide (PPO), poly paraphenylene terephthalamide (for example, KEVLAR^{®}), polysulfone, nylon, nylon-12 (such as GRILAMID^{®} available from EMS American Grilon), perfluoro(propyl vinyl ether) (PFA), ethylene vinyl alcohol, polyolefin, polystyrene, epoxy, polyvinylidene chloride (PVdC), poly(styrene-b-isobutylene-b-styrene) (for example, SIBS and/or SIBS A), polycarbonates, ionomers, biocompatible polymers, other suitable materials, or mixtures, combinations, copolymers thereof, polymer/metal composites, and the like. In some embodiments the sheath can be blended with a liquid crystal polymer (LCP). For example, the mixture can contain up to about 6 percent LCP.

In at least some embodiments, portions or all of the pump/catheter assembly and its related components may be doped with, made of, or otherwise include a radiopaque material. Radiopaque materials are understood to be materials capable of producing a relatively bright image on a fluoroscopy screen or another imaging technique during a medical procedure. This relatively bright image aids the user of the pump/catheter assembly and its related components in determining its location. Some examples of radiopaque materials can include, but are not limited to, gold, platinum, palladium, tantalum, tungsten alloy, polymer material loaded with a radiopaque filler, and the like. Additionally, other radiopaque marker bands and/or coils may also be incorporated into the design of the pump/catheter assembly and its related components to achieve the same result.

It should be understood that this disclosure is, in many respects, only illustrative. Changes may be made in details, particularly in matters of shape, size, and arrangement of steps without exceeding the scope of the disclosure. This may include, to the extent that it is appropriate, the use of any of the features of one example embodiment being used in other embodiments. The invention is defined by the following claims.

## Claims

1. A thrombectomy catheter and pump assembly (14), the assembly comprising:
a thrombectomy catheter (58);
a pump (56);
a connection manifold assembly (62) positioned between the thrombectomy catheter and the pump;
an effluent return tube (66) fluidly coupled to the thrombectomy catheter and the connection manifold assembly;
an effluent collection bag (28);
an effluent waste tube (68) fluidly coupled to the connection manifold assembly and the effluent collection bag; **characterised by**
a valve (400, 600, 700, 800, 900) positioned in line with the effluent waste tube; and
a fluid inlet tube (490) fluidly coupled to a first end (402) of the valve.

2. The assembly of claim 1, wherein the valve (400, 600, 700, 800, 900) is configured to selectively allow a flow of effluent from the thrombectomy catheter (58) to the effluent collection bag (28) during use of the thrombectomy catheter.

3. The assembly of any one of claims 1-2, wherein the valve is a spool valve (400, 600, 700, 800, 900) comprising a valve body (410, 610, 710, 810, 910) defining an interior lumen (460, 626, 726, 826, 926) and a spool (452, 634, 734, 834, 934) movably disposed within the interior lumen.

4. The assembly of claim 3, wherein the spool (452, 634, 734, 834, 934) further comprises an annular groove (456, 654, 754, 854, 954) extending about a circumference of the spool, the annular groove positioned between a first end (646, 746, 846, 946) of the spool and a second end (650, 750, 850, 950) of the spool.

5. The assembly of claim 3 or 4, wherein the spool valve (400, 600, 700, 800, 900) includes an effluent inlet port (408, 608, 708, 808, 908) fluidly coupled to the effluent waste tube (68) downstream of the connection manifold assembly (62) and an effluent outlet port (412, 612, 712, 812, 912) fluidly coupled to the effluent waste tube upstream of the effluent collection bag (28).

6. The assembly of claim 5, wherein the effluent inlet port (408, 608, 708, 808, 908) and the effluent outlet port (412, 612, 712, 812, 912) are positioned on opposite sides of the valve body (410, 610, 710, 810, 910); or extend at a generally orthogonal angle to one another.

7. The assembly of claim 5, wherein the spool valve (600, 700, 800, 900) includes a fluid inlet port (606, 706, 806, 906) and an aperture (660, 760, 860, 960) extending through a wall (658, 758, 858, 958) of the spool (634, 734, 834, 934) such that the fluid inlet port is in communication with either the effluent outlet port (612, 712, 812) or a fluid outlet port (990).

8. The assembly of any one of claims 5 to 7, further comprising first and second O-rings (640, 740, 840, 940) positioned between an outer surface (642, 742, 842, 942) of the spool (634, 734, 834, 934) and an inner surface (644, 744, 844, 944) of the valve body (610, 710, 810, 910), wherein when the valve (600, 700, 800, 900) is in a closed configuration the effluent inlet port (608, 708, 808, 908) is positioned between the first and second O-rings.

9. The assembly of any one of claims 5-8, wherein the effluent inlet port (408, 608, 708, 808, 908) and the effluent outlet port (412, 612, 712, 812, 912) are in fluid communication with the interior lumen (460, 626, 726, 826, 926) of the valve body (410, 610, 710, 810, 910) when the valve (400, 600, 700, 800, 900) is in an open position, and wherein the effluent inlet port and the effluent outlet port are fluidly isolated from the interior lumen of the valve body when the valve is in a closed position.

10. The assembly of claim 9, wherein the effluent inlet port (408, 608, 708, 808, 908) and the effluent outlet port (412, 612, 712, 812, 912) are in fluid communication with the interior lumen (460, 626, 726, 826, 926) of the valve body (410, 610, 710, 810, 910) when the annular groove (456, 654, 754, 854, 954) of the spool (452, 634, 734, 834, 934) is aligned with the effluent inlet port and the effluent outlet port.

11. The assembly of any one of claims 3-10, wherein the spool valve (400, 600, 700, 800, 900) includes a fluid inlet port (406, 606, 706, 806, 906) and a fluid inlet tube (490), the fluid inlet tube extending from a first end (492) fluidly coupled to the fluid inlet port to a second end (493) configured to be fluidly coupled to a fluid source.

12. The assembly of claim 11, wherein an intermediate portion (494) of the fluid inlet tube (490) is configured to be positioned within a roller pump (240) of a thrombectomy catheter system drive unit,
wherein a flow of fluid through the fluid inlet tube is configured to move the spool (452, 634, 734, 834, 934) from a first closed configuration to a second open configuration;
wherein when the spool is in the second open configuration, effluent flows freely from the thrombectomy catheter (58) to the effluent collection bag (28);
wherein when the spool is in the first closed configuration, effluent flow from the thrombectomy catheter to the effluent collection bag is precluded.

13. A thrombectomy catheter and pump assembly (14), the assembly comprising:
a thrombectomy catheter (58);
a pump (56);
a connection manifold assembly (62) positioned between the thrombectomy catheter and the pump;
an effluent return tube (66) fluidly coupled to the thrombectomy catheter and the connection manifold assembly;
an effluent collection bag (28);
an effluent waste tube (68) fluidly coupled to the connection manifold assembly and the effluent collection bag; **characterised by**
a spool valve (400) positioned in line with the effluent waste tube, the spool valve comprising:
a valve body (410);
an effluent inlet port (408) in fluid communication with a first portion of the effluent waste tube;
an effluent outlet port (412) in fluid communication with a second portion of the effluent waste tube;
a fluid inlet port (406), the fluid inlet port at least substantially fluidly isolated from the effluent inlet port and the effluent outlet port; and
a spool (452) movably disposed within an interior lumen (460) of the valve body;
wherein the spool is configured to move between a closed configuration configured to fluidly isolate the effluent inlet port and the effluent outlet port from one another and an open configuration configured to fluidly couple the effluent inlet port and the effluent outlet port.

14. The assembly of claim 12, wherein a flow of fluid into the fluid inlet port (406) during operation of the thrombectomy catheter is configured to move the spool (452) from the closed configuration to the open configuration.

15. The assembly of claim 12, wherein when the spool (452) is in the open configuration effluent flows freely from the thrombectomy catheter (58) to the effluent collection bag (28).

## Patentansprüche

1. Thrombektomiekatheter- und Pumpenanordnung (14), wobei die Anordnung aufweist:
einen Thrombektomiekatheter (58);
eine Pumpe (56);
eine Verbindungsverteileranordnung (62), die zwischen dem Thrombektomiekatheter und der Pumpe positioniert ist;
einen Effluent-Rücklaufschlauch (66), der fluidisch mit dem Thrombektomiekatheter und der Verbindungsverteileranordnung gekoppelt ist;
einen Effluent-Sammelbeutel (28);
einen Effluent-Abfallschlauch (68), der fluidisch mit der Verbindungsverteileranordnung und dem Effluent-Sammelbeutel gekoppelt ist;
**gekennzeichnet durch**
ein Ventil (400, 600, 700, 800, 900), das in Reihe mit dem Effluent-Abfallschlauch angeordnet ist; und
einem Fluideinlassschlauch (490), der fluidisch mit einem ersten Ende (402) des Ventils gekoppelt ist.

2. Anordnung nach Anspruch 1, wobei das Ventil (400, 600, 700, 800, 900) konfiguriert ist, selektiv einen Effluent-Fluss vom Thrombektomiekatheter (58) zum Effluent-Sammelbeutel (28) während einer Verwendung des Thrombektomiekatheters zu ermöglichen.

3. Anordnung nach einem der Ansprüche 1 bis 2, wobei das Ventil ein Schieberventil (400, 600, 700, 800, 900) ist, das einen Ventilkörper (410, 610, 710, 810, 910), der ein inneres Lumen (460, 626, 726, 826, 926) definiert, und einen Schieber (452, 634, 734, 834, 934) aufweist, der beweglich innerhalb des inneren Lumens angeordnet ist.

4. Anordnung nach Anspruch 3, wobei der Schieber (452, 634, 734, 834, 934) ferner eine ringförmige Nut (456, 654, 754, 854, 954) aufweist, die sich um einen Umfang des Schiebers erstreckt, wobei die ringförmige Nut zwischen einem ersten Ende (646, 746, 846, 946) des Schiebers und einem zweiten Ende (650, 750, 850, 950) des Schiebers angeordnet ist.

5. Anordnung nach Anspruch 3 oder 4, wobei das Schieberventil (400, 600, 700, 800, 900) einen Effluent-Einlassanschluss (408, 608, 708, 808, 908), der fluidisch mit dem Effluent-Abfallschlauch (68) stromabwärts der Verbindungsverteileranordnung (62) gekoppelt ist, und einen Effluent-Auslassanschluss (412, 612, 712, 812, 912) aufweist, der fluidisch mit dem Effluent-Abfallschlauch stromaufwärts des Effluent-Sammelbeutels (28) gekoppelt ist.

6. Anordnung nach Anspruch 5, wobei der Effluent-Einlassanschluss (408, 608, 708, 808, 908) und der Effluent-Auslassanschluss (412, 612, 712, 812, 912) auf gegenüberliegenden Seiten des Ventilkörpers (410, 610, 710, 810, 910) angeordnet sind; oder sich in einem im Wesentlichen orthogonalen Winkel zueinander erstrecken.

7. Anordnung nach Anspruch 5, wobei das Schieberventil (600, 700, 800, 900) einen Fluideinlassanschluss (606, 706, 806, 906) und eine Öffnung (660, 760, 860, 960) aufweist, die sich durch eine Wand (658, 758, 858, 958) des Schiebers (634, 734, 834, 934) erstreckt, so dass der Fluideinlassanschluss entweder mit dem Effluent-Auslassanschluss (612, 712, 812) oder mit einem Fluidauslassanschluss (990) in Verbindung steht.

8. Anordnung nach einem der Ansprüche 5 bis 7, die ferner einen ersten und einen zweiten O-Ring (640, 740, 840, 940) aufweist, die zwischen einer Außenfläche (642, 742, 842, 942) des Schiebers (634, 734, 834, 934) und einer Innenfläche (644, 744, 844, 944) des Ventilkörpers (610, 710, 810, 910) angeordnet sind, wobei, wenn sich das Ventil (600, 700, 800, 900) in einer geschlossenen Konfiguration befindet, der Effluent-Einlassanschluss (608, 708, 808, 908) zwischen dem ersten und dem zweiten O-Ring positioniert ist.

9. Anordnung nach einem der Ansprüche 5 bis 8, wobei der Effluent-Einlassanschluss (408, 608, 708, 808, 908) und der Effluent-Auslassanschluss (412, 612, 712, 812, 912) in Fluidverbindung mit dem inneren Lumen (460, 626, 726, 826, 926) des Ventilkörpers (410, 610, 710, 810, 910) stehen, wenn sich das Ventil (400, 600, 700, 800, 900) in einer offenen Position befindet, und wobei der Effluent-Einlassanschluss und der Effluent-Auslassanschluss vom inneren Lumen des Ventilkörpers fluidisch isoliert sind, wenn sich das Ventil in einer geschlossenen Position befindet.

10. Anordnung nach Anspruch 9, wobei der Effluent-Einlassanschluss (408, 608, 708, 808, 908) und der Effluent-Auslassanschluss (412, 612, 712, 812, 912) in Fluidverbindung mit dem inneren Lumen (460, 626, 726, 826, 926) des Ventilkörpers (410, 610, 710, 810, 910) stehen, wenn die ringförmige Nut (456, 654, 754, 854, 954) des Schiebers (452, 634, 734, 834, 934) mit dem Effluent-Einlassanschluss und dem Effluent-Auslassanschluss ausgerichtet ist.

11. Anordnung nach einem der Ansprüche 3 bis 10, wobei das Schieberventil (400, 600, 700, 800, 900) einen Fluideinlassanschluss (406, 606, 706, 806, 906) und einen Fluideinlassschlauch (490) aufweist, wobei sich der Fluideinlassschlauch von einem ersten Ende (492), das fluidisch mit dem Fluideinlassanschluss gekoppelt ist, zu einem zweiten Ende (493) erstreckt, das konfiguriert ist, fluidisch mit einer Fluidquelle gekoppelt zu werden.

12. Anordnung nach Anspruch 11, wobei ein Zwischenabschnitt (494) des Fluideinlassschlauchs (490) konfiguriert ist, innerhalb einer Rollenpumpe (240) einer Thrombektomiekathetersystem-Antriebseinheit positioniert zu werden,
wobei ein Fluidfluss durch den Fluideinlassschlauch konfiguriert ist, den Schieber (452, 634, 734, 834, 934) aus einer ersten geschlossenen Konfiguration in eine zweite offene Konfiguration zu bewegen;
wobei, wenn sich der Schieber in der zweiten offenen Konfiguration befindet, ein Effluent frei vom Thrombektomiekatheter (58) zum Effluent-Sammelbeutel (28) fließt;
wobei, wenn sich der Schieber in der ersten geschlossenen Konfiguration befindet, ein Effluent-Fluss vom Thrombektomiekatheter zum Effluent-Sammelbeutel verhindert wird.

13. Thrombektomiekatheter- und Pumpenanordnung (14), wobei die Anordnung aufweist:
einen Thrombektomiekatheter (58);
eine Pumpe (56);
eine Verbindungsverteileranordnung (62), die zwischen dem Thrombektomiekatheter und der Pumpe positioniert ist;
einen Effluent-Rücklaufschlauch (66), der fluidisch mit dem Thrombektomiekatheter und der Verbindungsverteileranordnung gekoppelt ist;
einen Effluent-Sammelbeutel (28);
einen Effluent-Abfallschlauch (68), der fluidisch mit der Verbindungsverteileranordnung und dem Effluent-Sammelbeutel gekoppelt ist;
**gekennzeichnet durch**
ein Schieberventil (400), das in Reihe mit dem Effluent-Abfallschlauch angeordnet ist,
wobei das Schieberventil aufweist:
einen Ventilkörper (410);
einen Effluent-Einlassanschluss (408), der in Fluidverbindung mit einem ersten Abschnitt des Effluent-Schlauchs steht;
einen Effluent-Auslassanschluss (412), der in Fluidverbindung mit einem zweiten Abschnitt des Effluent-Schlauchs steht;
einen Fluideinlassanschluss (406), wobei der Fluideinlassanschluss mindestens im Wesentlichen fluidisch von dem Effluent-Einlassanschluss und dem Effluent-Auslassanschluss isoliert ist; und
einen Schieber (452), der beweglich in einem inneren Lumen (460) des Ventilkörpers angeordnet ist;
wobei der Schieber konfiguriert ist, sich zwischen einer geschlossenen Konfiguration, die konfiguriert ist, den Effluent-Einlassanschluss und den Effluent-Auslassanschluss fluidisch voneinander zu isolieren, und einer offenen Konfiguration zu bewegen, die konfiguriert ist, den Effluent-Einlassanschluss und den Effluent-Auslassanschluss fluidisch zu koppeln.

14. Anordnung nach Anspruch 12, wobei ein Fluidfluss in den Fluideinlassanschluss (406) während eines Betriebs des Thrombektomiekatheters konfiguriert ist, den Schieber (452) von der geschlossenen Konfiguration in die offene Konfiguration zu bewegen.

15. Anordnung nach Anspruch 12, wobei, wenn sich der Schieber (452) in der offenen Konfiguration befindet, ein Effluent frei aus dem Thrombektomiekatheter (58) in den Effluent-Sammelbeutel (28) fließt.

## Revendications

1. Ensemble cathéter de thrombectomie et pompe (14), l'ensemble comprenant :
un cathéter de thrombectomie (58) ;
une pompe (56) ;
un ensemble collecteur de raccordement (62) positionné entre le cathéter de thrombectomie et la pompe ;
un tube de retour d'effluent (66) couplé de manière fluidique au cathéter de thrombectomie et à l'ensemble collecteur de raccordement ;
un sac de collecte d'effluent (28) ;
un tube d'évacuation d'effluent (68) couplé de manière fluidique à l'ensemble collecteur de raccordement et au sac de collecte d'effluent ;
**caractérisé par** une valve (400, 600, 700, 800, 900) positionnée en ligne avec le tube d'évacuation d'effluent ; et
un tube d'entrée de fluide (490) couplé de manière fluidique à une première extrémité (402) de la valve.

2. Ensemble selon la revendication 1, dans lequel la valve (400, 600, 700, 800, 900) est configurée pour permettre sélectivement un écoulement d'effluent du cathéter de thrombectomie (58) vers le sac de collecte d'effluent (28) lors de l'utilisation du cathéter de thrombectomie.

3. Ensemble selon l'une quelconque des revendications 1 à 2, dans lequel la valve est une valve à tiroir (400, 600, 700, 800, 900) comprenant un corps de valve (410, 610, 710, 810, 910) définissant une lumière intérieure (460, 626, 726, 826, 926) et un tiroir (452, 634, 734, 834, 934) disposé de manière mobile à l'intérieur de la lumière intérieure.

4. Ensemble selon la revendication 3, dans lequel le tiroir (452, 634, 734, 834, 934) comprend en outre une gorge annulaire (456, 654, 754, 854, 954) s'étendant autour d'une circonférence du tiroir, la gorge annulaire étant positionnée entre une première extrémité (646, 746, 846, 946) du tiroir et une deuxième extrémité (650, 750, 850, 950) du tiroir.

5. Ensemble selon la revendication 3 ou 4, dans lequel la valve à tiroir (400, 600, 700, 800, 900) comprend un orifice d'entrée d'effluent (408, 608, 708, 808, 908) couplé de manière fluidique au tube d'évacuation d'effluent (68) en aval de l'ensemble collecteur de raccordement (62) et un orifice de sortie d'effluent (412, 612, 712, 812, 912) couplé de manière fluidique au tube d'évacuation d'effluent en amont du sac de collecte d'effluent (28).

6. Ensemble selon la revendication 5, dans lequel l'orifice d'entrée d'effluent (408, 608, 708, 808, 908) et l'orifice de sortie d'effluent (412, 612, 712, 812, 912) sont positionnés sur des côtés opposés du corps de valve (410, 610, 710, 810, 910) ; ou s'étendent selon un angle généralement orthogonal l'un par rapport à l'autre.

7. Ensemble selon la revendication 5, dans lequel la valve à tiroir (600, 700, 800, 900) comprend un orifice d'entrée de fluide (606, 706, 806, 906) et une ouverture (660, 760, 860, 960) s'étendant à travers une paroi (658, 758, 858, 958) du tiroir (634, 734, 834, 934) de sorte que l'orifice d'entrée de fluide soit en communication avec l'orifice de sortie d'effluent (612, 712, 812) ou un orifice de sortie de fluide (990).

8. Ensemble selon l'une quelconque des revendications 5 à 7, comprenant en outre un premier et un deuxième joint torique (640, 740, 840, 940) positionnés entre une surface externe (642, 742, 842, 942) du tiroir (634, 734, 834, 934) et une surface interne (644, 744, 844, 944) du corps de valve (610, 710, 810, 910), dans lequel, lorsque la valve (600, 700, 800, 900) est dans une configuration fermée, l'orifice d'entrée d'effluent (608, 708, 808, 908) est positionné entre le premier et le deuxième joint torique.

9. Ensemble selon l'une quelconque des revendications 5 à 8, dans lequel l'orifice d'entrée d'effluent (408, 608, 708, 808, 908) et l'orifice de sortie d'effluent (412, 612, 712, 812, 912) sont en communication fluidique avec la lumière intérieure (460, 626, 726, 826, 926) du corps de valve (410, 610, 710, 810, 910) lorsque la valve (400, 600, 700, 800, 900) est dans une position ouverte, et dans lequel l'orifice d'entrée d'effluent et l'orifice de sortie d'effluent sont isolés de manière fluidique de la lumière intérieure du corps de valve lorsque la valve est dans une position fermée.

10. Ensemble selon la revendication 9, dans lequel l'orifice d'entrée d'effluent (408, 608, 708, 808, 908) et l'orifice de sortie d'effluent (412, 612, 712, 812, 912) sont en communication fluidique avec la lumière intérieure (460, 626, 726, 826, 926) du corps de valve (410, 610, 710, 810, 910) lorsque la gorge annulaire (456, 654, 754, 854, 954) du tiroir (452, 634, 734, 834, 934) est alignée avec l'orifice d'entrée d'effluent et l'orifice de sortie d'effluent.

11. Ensemble selon l'une quelconque des revendications 3 à 10, dans lequel la valve à tiroir (400, 600, 700, 800, 900) comprend un orifice d'entrée de fluide (406, 606, 706, 806, 906) et un tube d'entrée de fluide (490), le tube d'entrée de fluide s'étendant d'une première extrémité (492) couplée de manière fluidique à l'orifice d'entrée de fluide à une deuxième extrémité (493) configurée pour être couplée de manière fluidique à une source de fluide.

12. Ensemble selon la revendication 11, dans lequel une partie intermédiaire (494) du tube d'entrée de fluide (490) est configurée pour être positionnée à l'intérieur d'une pompe à galets (240) d'une unité d'entraînement de système de cathéter de thrombectomie,
dans lequel un écoulement de fluide à travers le tube d'entrée de fluide est configuré pour déplacer le tiroir (452, 634, 734, 834, 934) d'une première configuration fermée à une deuxième configuration ouverte ;
dans lequel, lorsque le tiroir est dans la deuxième configuration ouverte, l'effluent s'écoule librement du cathéter de thrombectomie (58) vers le sac de collecte d'effluent (28) ;
dans lequel, lorsque le tiroir est dans la première configuration fermée, l'écoulement d'effluent du cathéter de thrombectomie vers le sac de collecte d'effluent est empêché.

13. Ensemble cathéter de thrombectomie et pompe (14), l'ensemble comprenant :
un cathéter de thrombectomie (58) ;
une pompe (56) ;
un ensemble collecteur de raccordement (62) positionné entre le cathéter de thrombectomie et la pompe ;
un tube de retour d'effluent (66) couplé de manière fluidique au cathéter de thrombectomie et à l'ensemble collecteur de raccordement ;
un sac de collecte d'effluent (28) ;
un tube d'évacuation d'effluent (68) couplé de manière fluidique à l'ensemble collecteur de raccordement et au sac de collecte d'effluent ;
**caractérisé par** une valve à tiroir (400) positionnée en ligne avec le tube d'évacuation d'effluent, la valve à tiroir comprenant :
un corps de valve (410) ;
un orifice d'entrée d'effluent (408) en communication fluidique avec une première partie du tube d'évacuation d'effluent ;
un orifice de sortie d'effluent (412) en communication fluidique avec une deuxième partie du tube d'évacuation d'effluent ;
un orifice d'entrée de fluide (406), l'orifice d'entrée de fluide étant au moins sensiblement isolé de manière fluidique de l'orifice d'entrée d'effluent et de l'orifice de sortie d'effluent ; et
un tiroir (452) disposé de manière mobile à l'intérieur d'une lumière interne (460) du corps de valve ;
le tiroir étant configuré pour se déplacer entre une configuration fermée configurée pour isoler de manière fluidique l'orifice d'entrée d'effluent et l'orifice de sortie d'effluent l'un de l'autre et une configuration ouverte configurée pour coupler de manière fluidique l'orifice d'entrée d'effluent et l'orifice de sortie d'effluent.

14. Ensemble selon la revendication 12, dans lequel un écoulement de fluide dans l'orifice d'entrée de fluide (406) lors du fonctionnement du cathéter de thrombectomie est configuré pour déplacer le tiroir (452) de la configuration fermée à la configuration ouverte.

15. Ensemble selon la revendication 12, dans lequel, lorsque le tiroir (452) est en configuration ouverte, l'effluent s'écoule librement du cathéter de thrombectomie (58) vers le sac de collecte d'effluent (28).
